(19) Europäisches Patentamt / European Patent Office / Office européen des brevets

(11) EP 2 945 602 B1

(12) **EUROPEAN PATENT SPECIFICATION**

(45) Date of publication and mention of the grant of the patent:
**10.01.2018 Bulletin 2018/02**

(21) Application number: **14700914.6**

(22) Date of filing: **20.01.2014**

(51) Int Cl.:
***A61K 8/41*** *(2006.01)* ***A61K 8/49*** *(2006.01)*
***A61Q 17/04*** *(2006.01)* ***A61K 8/29*** *(2006.01)*
***A61Q 19/08*** *(2006.01)*

(86) International application number:
**PCT/EP2014/051013**

(87) International publication number:
**WO 2014/111566 (24.07.2014 Gazette 2014/30)**

(54) **COSMETIC OR DERMATOLOGICAL COMPOSITION COMPRISING A MEROCYANINE AND AN INSOLUBLE ORGANIC UV-SCREENING AGENT AND/OR AN INSOLUBLE INORGANIC UV-SCREENING AGENT**

KOSMETISCHE ODER DERMATOLOGISCHE ZUSAMMENSETZUNG MIT EINEM MEROCYANIN UND EINEM UNLÖSLICHEN ORGANISCHEN UV-ABSCHIRMENDEN UND/ODER EINEM UNLÖSLICHEN ANORGANISCHEN UV-ABSCHIRMENDEM MITTEL

COMPOSITION COSMÉTIQUE OU DERMATOLOGIQUE COMPRENANT UNE MÉROCYANINE ET UN AGENT ANTI-UV ORGANIQUE INSOLUBLE ET/OU UN AGENT ANTI-UV INORGANIQUE INSOLUBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:
**21.01.2013 FR 1350483**
**21.01.2013 FR 1350488**
**20.02.2013 US 201361767042 P**
**20.02.2013 US 201361767031 P**

(43) Date of publication of application:
**25.11.2015 Bulletin 2015/48**

(73) Proprietor: **L'Oréal**
**75008 Paris (FR)**

(72) Inventors:
- **ROUDOT, Angelina**
**F-94270 Le Kremlin Bicêtre (FR)**
- **CANDAU, Didier**
**F-91570 Bièvres (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**EP-A1- 0 518 773** **WO-A1-2011/113718**
**WO-A1-2013/010590** **WO-A2-2008/090066**
**WO-A2-2009/027258** **WO-A2-2013/011094**

- **"Mixtures comprising Cinnamates, Benzotriazoles and Merocyanines", IP.COM JOURNAL, IP.COM INC., WEST HENRIETTA, NY, US, 12 November 2009 (2009-11-12), XP013135311, ISSN: 1533-0001**
- **"Process for producing 3-amino-2-cyclohexan-1-ylidene compounds", IP.COM JOURNAL, IP.COM INC., WEST HENRIETTA, NY, US, 29 April 2009 (2009-04-29), XP013131313, ISSN: 1533-0001**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 945 602 B1

## Description

The present invention relates to a cosmetic or dermatological composition comprising, in a physiologically acceptable support:

a) at least one oily phase and
b) at least one merocyanine compound of formula (1) defined herein below and
c) at least one insoluble organic UV-screening agent and/or one insoluble inorganic UV-screening agent.

The present invention also relates to a non-therapeutic cosmetic process for caring for and/or making up a keratin material, comprising the application, to the surface of the said keratin material, of at least one composition according to the invention as defined above.

The invention also relates to a non-therapeutic cosmetic process for limiting the darkening of the skin and/or improving the colour and/or uniformity of the complexion, comprising the application, to the surface of the keratin material, of at least one composition as defined previously.

The invention also relates to a non-therapeutic cosmetic process for preventing and/or treating the signs of ageing of a keratin material, comprising the application, to the surface of the keratin material, of at least one composition as defined previously.

It is known that radiation with wavelengths of between 280 nm and 400 nm permits tanning of the human epidermis and that radiation with wavelengths of between 280 and 320 nm, known as UV-B rays, harms the development of a natural tan. Exposure is also liable to bring about a detrimental change in the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UV-A rays, even of short duration, under normal conditions can result in damage to the collagen fibres and the elastin, which is reflected by a modification in the microrelief of the skin, the appearance of wrinkles and uneven pigmentation (brown spots, lack of uniformity of the complexion).

Protection against UVA and UVB rays is thus necessary. An efficient photoprotective product should protect against both UVA and UVB radiation.

Many photoprotective compositions have been proposed to date to overcome the effects induced by UVA and/or UVB radiation. They generally contain organic and/or inorganic UV-screening agents, which function according to their own chemical nature and according to their own properties by absorption, reflection or scattering of the UV radiation. They generally contain mixtures of liposoluble organic screening agents and/or water-soluble UV-screening agents in combination with metal oxide pigments, such as titanium dioxide or zinc oxide.

Many cosmetic compositions for limiting the darkening of the skin and improving the colour and uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already-existing melanin leading to darkening of the skin colour.

However, it is extremely difficult to find a composition which contains a particular combination of UV-screening agents that would be especially suited to improving the quality of the skin as regards both the colour and its mechanical elasticity properties. This improvement is particularly sought on already-pigmented skin so as not to increase the melanin pigmentary load or the structure of the melanin already present in the skin.

In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability alone or in combination with other UV-screening agents. They should also be colourless or at least have a colour that is cosmetically acceptable to the consumer.

One of the main drawbacks known to date of these antisun compositions is that their systems for screening out UVA and UVB radiation are insufficiently effective against UV rays and in particular against long UVA rays with wavelengths beyond 370 nm, for the purpose of controlling photo-induced pigmentation and its evolution by a UV-screening system on the entire UV spectrum.

In this respect, a particularly advantageous family of UV-A screening agents is currently composed of dibenzoylmethane derivatives and in particular 4-tert-butyl-4'-methoxydibenzoylmethane, this being because they have high intrinsic absorbing power. These dibenzoylmethane derivatives, which are products that are now well known per se as screening agents that are active in the UV-A range, are described in particular in French patent applications FR-A-2 326

405 and FR-A-2 440 933, and also in European patent application EP-A-0 114 607; 4-tert-butyl-4'-methoxydibenzoylmethane is moreover currently sold under the trade name Parsol 1789® by the company DSM Nutritional Products. However, these compounds are photo-unstable and cannot be combined with UVB-screening agents to afford broad UV protection over the range 280 to 400 nm. In point of fact, these UVA and UVB screening systems thus constituted make it possible to protect from 280 nm to 370 nm, and at best to 380 nm in large amounts. Furthermore, they have the drawback of needing to be dissolved in substantial amounts of oil, which often leads to formulation difficulties and also to formulation sensory drawbacks, such as a greasy effect on application.

Insoluble organic screening agents thus appear to be particularly advantageous since they can be dispersed in water and introduced directly into the aqueous phase. The ones mentioned in patent US 5 869 030 are especially known. Among these screening agents, mention may be made most particularly of methylenebis(benzotriazolyl)tetramethylbutylphenol sold in solid form under the trade name Mixxim BB/100® by Fairmount Chemical, or in micronized form as an aqueous dispersion under the trade name Tinosorb M® by the company BASF. This UV-screening agent absorbs both UVA radiation and UVB radiation.

The UVA and UVB screening systems described in the said patent US 5 869 030 especially with the UVB and UVA screening agent methylenebis(benzotriazolyl)tetramethylbutylphenol do not, however, make it possible to afford broad UV protection over the range 280 to 400 nm especially having an observable absorbance up to a wavelength of 400 nm inclusive.

The insoluble inorganic UV-screening agents are generally metal oxide pigments with a mean elemental particle size of less than or equal to 0.5 μm, more preferentially between 0.005 and 0.5 μm, even more preferentially between 0.01 and 0.2 μm, better still between 0.01 and 0.1 μm and more particularly preferentially between 0.015 and 0.05 μm. They may be selected in particular from titanium oxide, zinc oxide, iron oxide, zirconium oxide and cerium oxide, or mixtures thereof. The agent most commonly used is titanium dioxide. Such metal oxide pigments have in particular been described in patent application EP-A-0 518 773.

These insoluble inorganic screening agents are particularly advantageous since they do not need to be dissolved. They may be dispersed and thus introduced directly into the aqueous or oily phase of the antisun formulations, especially oil-in-water emulsions (i.e. a cosmetically and/or dermatologically acceptable support consisting of an aqueous dispersing continuous phase and of a fatty dispersed discontinuous phase) or water-in-oil emulsions (aqueous phase dispersed in a continuous fatty phase).

However, UVA and UVB screening systems containing these inorganic screening agents do not make it possible to afford broad UV protection over the range 280 to 400 nm especially having an observable absorbance up to a wavelength of 400 nm inclusive.

Merocyanine compounds are known in patent US 4 195 999, patent application WO 2004/006 878, patent applications WO2008/090066, WO2011/113718, WO2009/027258, WO2013/010590, WO2013/011094, WO20130/11480 and the documents IP COM JOURNAL N°000179675D published on February 23, 2009, IP COM JOURNAL N°000182396D published on April 29, IP COM JOURNAL N° 000189542D published on November 12, 2009, IP COM Journal N°IPCOM000011179D published on 03/04/2004.

Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

The UVA and UVB screening systems consisting of some of these merocyanine screening agents as the compound Octyl-5-N,N-diethylamino-2-phenysulfonyl-2,4-pentadienoate corresponding to the compound MC172 of structure

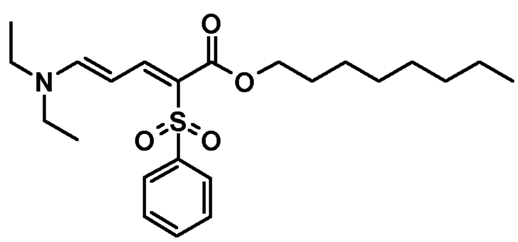

and insoluble organic filters and/or insoluble inorganic filters do not always make it possible to afford broad UV protection over the range 280 to 400 nm and especially to obtain an observable absorbance up to a wavelength of 400 nm inclusive.

There thus remains a need to find a novel UVA and UVB screening system based on a merocyanine compound and an insoluble organic UV-screening agent and/or an insoluble inorganic UV-screening agent which is photostable and which ensures overall protection against UV rays from 280 to 400 nm especially having notable absorbance ranging up to a wavelength of 400 nm inclusive, without the drawbacks as previously defined.

The Applicant has discovered, surprisingly, that this objective can be achieved by using at least one insoluble organic and/or at least one insoluble inorganic UV-screening agent and at least one particular merocyanine of formula (1) which will be defined in greater detail hereinbelow.

Furthermore, the merocyanine compounds of formula (1) herein below, present surprinsingly the advantage to be significantly less colored than the merocyanine compounds as disclosed in the application WO2008/090066 as the compound MC11 also called MC03 in the application WO2009/027258.

Those discoveries form the basis of the present invention.

Thus, in accordance with one of the objects of the present invention, a cosmetic or dermatological composition is now proposed, comprising, in a physiologically acceptable support:

a) at least one oily phase and
b) at least one merocyanine compound of formula (1) defined herein below and
c) at least one insoluble organic UV-screening agent and/or one insoluble inorganic UV-screening agent.

The present invention also relates to a non-therapeutic cosmetic process for caring for and/or making up a keratin material, comprising the application to the surface of the said keratin material of at least one composition according to the invention as defined above.

The invention also relates to a non-therapeutic cosmetic process for limiting the darkening of the skin and/or improving the colour and/or the uniformity of the complexion, comprising the application, to the surface of the keratin material, of at least one composition as defined previously.

The invention also relates to a non-therapeutic cosmetic process for preventing and/or treating the signs of ageing of a keratin material, comprising the application to the surface of the keratin material of at least one composition as defined previously.

Other characteristics, aspects and advantages of the invention will emerge on reading the detailed description that follows.

The expression "human keratin materials" means the skin (body, face, area around the eyes), hair, eyelashes, eyebrows, body hair, nails, lips or mucous membranes.

The term "physiologically acceptable" means compatible with the skin and/or its integuments, having a pleasant colour, odour and feel and not causing any unacceptable discomfort (stinging, tautness or redness) liable to discourage the consumer from using this composition.

The term "insoluble UV-screening agent" means any cosmetic or dermatological organic or mineral compound for screening out UV radiation which has a solubility in water of less than 0.5% by weight and a solubility of less than 0.5% by weight in the majority of organic solvents such as liquid paraffin, fatty alcohol benzoates and fatty acid triglycerides, for example Miglyol® 812 sold by the company Dynamit Nobel. This solubility, determined at 70°C, is defined as the amount of product in solution in the solvent at equilibrium with an excess of solid in suspension after returning to room temperature. It may be readily evaluated in the laboratory.

The term "organic UV-screening agent" means any organic chemical molecule that is capable of absorbing and/or of physically blocking (especially by reflection or scattering) UV radiation in the wavelength range between 280 and 400 nm.

The term "mineral UV-screening agent" means any non-organic chemical molecule that is capable of absorbing and/or of physically blocking (especially by reflection or scattering) UV radiation in the wavelength range between 280 and 400 nm.

The term "between X and Y" means the range of values also including the limits X and Y.

According to the invention, the term "preventing" or "prevention" means reducing the risk of occurrence or slowing down the occurrence of a given phenomenon, namely, according to the present invention, the signs of ageing of a keratin material.

**MEROCYANINES**

According to the present invention, the merocyanine compounds in accordance with the invention correspond to formula (1) below, and also the E/E- or E/Z-geometrical isomer forms thereof:

(1) 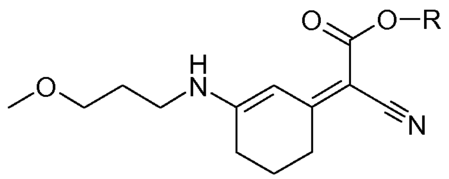

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being substituted with one or more O.

The merocyanine compounds of the invention may be in their E/E- or E/Z-geometrical isomer forms.

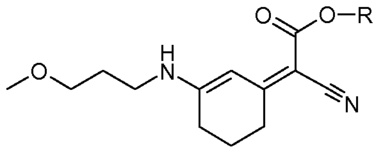

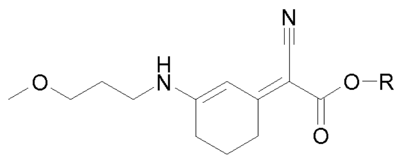

The preferential compounds of formula (1) are those in which:

R is a $C_1$-$C_{22}$ alkyl, which may be substituted with one or more O.

Among the compounds of formula (1), use will be made more particularly of those chosen from the following compounds, and also the E/E- or E/Z- geometrical isomer forms thereof:

| | | | |
|---|---|---|---|
| 1 | 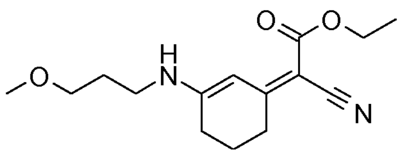 ethyl (2Z)-cyano{3-[(3-methoxypropyl)amino] cyclohex-2-en-1-ylidene}ethanoate | 4 | 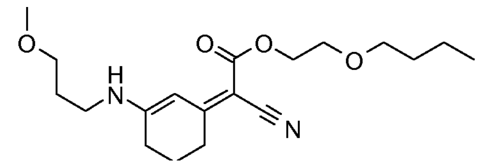 2-butoxyethyl (2Z)-cyano{3-[(3-methoxypropyl) amino]cyclohex-2-en-1-ylidene}ethanoate |
| 2 | 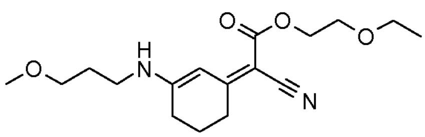 2-ethoxyethyl (2Z)-cyano{3-[(3-methoxypropyl) amino]cyclohex-2-en-1-ylidene}ethanoate | 5 | 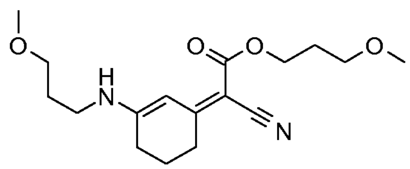 3-methoxypropyl (2Z)-cyano{3-[(3-methoxypropyl) amino]cyclohex-2-en-1-ylidene}ethanoate |
| 3 | 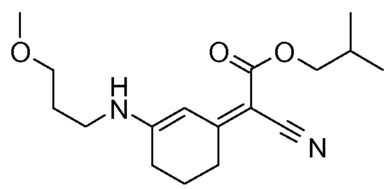 2-methylpropyl (2Z)-cyano{3-[(3-methoxypropyl) amino]cyclohex-2-en-1-ylidene}ethanoate | 6 | 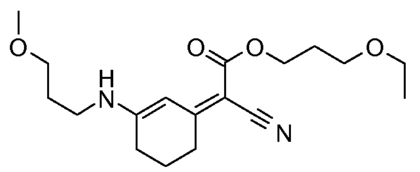 3-ethoxypropyl (2Z)-cyano{3-[(3-methoxypropyl) amino]cyclohex-2-en-1-ylidene}ethanoate |

According to a more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure:

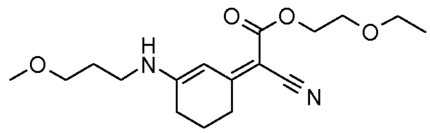

and/or in its E/E geometrical configuration having the following structure:

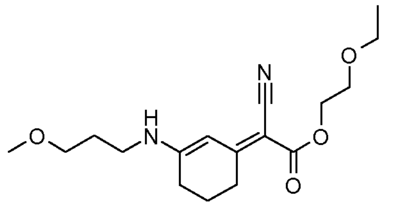

The merocyanines of formula (1) according to the invention are preferably present in the compositions according to the invention in a concentration ranging from 0.1% to 10% by weight and preferentially from 0.2% to 5% by weight relative to the total weight of the composition.

The compounds of formula (1) may be prepared according to the protocols described in Pat. Appl. WO 2007/071 582, in IP.com Journal (2009), 9(5A), 29-30 IPCOM000182396D under the title "Process for producing 3-amino-2-cyclohexan-1-ylidene compounds" and in US-A-4 749 643 on column 13, line 66 - column 14, line 57 and the references cited in this regard.

## INSOLUBLE ORGANIC UV-SCREENING AGENT

The insoluble organic UV-screening agents according to the invention preferably have a mean particle size which ranges from 0.01 to 5 μm, more preferentially from 0.01 to 2 μm and more particularly from 0.020 to 2 μm.

The mean particle diameter is measured with a particle size distribution analyser such as the Coulter N4 Plus machine manufactured by Beckman Coulter Inc.

The insoluble organic screening agents according to the invention may be brought to the desired particulate form by any suitable means, especially such as dry milling or milling in a solvent medium, screening, atomization, micronization or spraying.

The insoluble organic screening agents according to the invention in micronized form may in particular be obtained by means of a process for milling an insoluble organic UV-screening agent in the form of coarse-sized particles in the presence of a suitable surfactant for improving the dispersion of the particles thus obtained in the cosmetic formulations.

An example of a process for the micronization of insoluble organic screening agents is described in applications GB-A-2 303 549 and EP-A-893119. The milling apparatus used according to those documents may be an air-jet mill, a ball mill, a vibration mill or a hammer mill, and preferably a mill with high stirring speed or an impact mill and more particularly a rotary ball mill, a vibration mill, a tube mill or a rod mill.

According to this particular process, use is made, as surfactants for milling the said screening agents, of alkylpolyglucosides of structure $C_nH_{2n+1}O(C_6H_{10}O_5)_xH$ in which n is an integer from 8 to 16 and x is the average degree of polymerisation of the $(C_6H_{10}O_5)$ unit and ranges from 1.4 to 1.6. They may be chosen from $C_1$-$C_{12}$ esters of a compound of structure $C_nH_{2n+1}O(C_6H_{10}O_5)_xH$ and more particularly an ester obtained by reacting a $C_1$-$C_{12}$ carboxylic acid, such as formic acid, acetic acid, propionic acid, butyric acid, sulfosuccinic acid, citric acid or tartaric acid, with one or more free OH functions on the glucoside $(C_6H_{10}O_5)$ unit. As alkylpolyglucoside, mention may be made in particular of decylglucoside.

The said surfactants are generally used at a concentration ranging from 1% to 50% by weight and more preferentially from 5% to 40% by weight relative to the insoluble screening agent in its micronized form.

The insoluble organic UV-screening agents in accordance with the invention may be chosen especially from organic UV-screening agents of the oxalanilide type, of the triazine type, of the benzotriazole type, of the vinylamide type, of the cinnamide type, of the type comprising one or more benzazole and/or benzofuran, benzothiophenene groups or of the indole type; of the aryl vinylene ketone type; of the phenylene bis-benzoxazinone derivative type, or of the amide, sulfonamide or acrylonitrile carbamate derivative type, or mixtures thereof.

In the sense in which it is used in the present invention, the term "benzazole" encompasses benzothiazoles, benzoxazoles and benzimidazoles.

A/ Oxalanides

Among the UV-screening agents of the oxalanilide type in accordance with the invention, mention may be made of those corresponding to the structure:

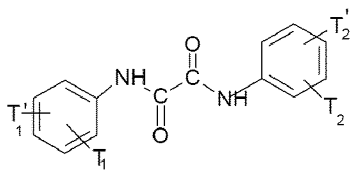

(I)

in which $T_1$, $T_1$', $T_2$ and $T_2$', which may be identical or different, denote a $C_1$-$C_8$ alkyl radical or a $C_1$-$C_8$ alkoxy radical. These compounds are described in patent application WO 95/22959.

Examples that may be mentioned include the commercial products Tinuvin® 315 and Tinuvin® 312 sold by the company BASF, having the respective structures:

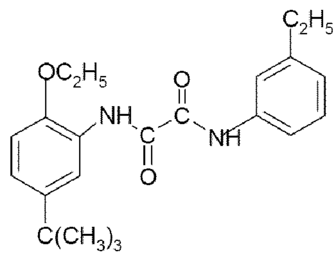

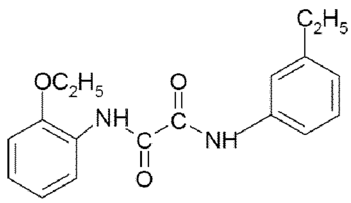

B/ Triazines

Among the insoluble UV-screening agents of the triazine type in accordance with the invention, mention may also be made of those corresponding to formula (II) below:

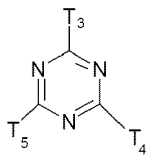

**(II)**

in which $T_3$, $T_4$ and $T_5$, independently, are phenyl, phenoxy or pyrrolo, in which the phenyl, phenoxy and pyrrolo are unsubstituted or substituted with one, two or three substituents chosen from OH, $C_1$-$C_{18}$alkyl or $C_1$-$C_{18}$alkoxy, $C_1$-$C_{18}$carboxyalkyl, $C_5$-$C_8$cycloalkyl, a methylbenzylidenecamphor group, a group - $(CH=CH)_n(CO)$-$OT_6$, with $T_6$ being either $C_1$-$C_{18}$alkyl or cinnamyl.

These compounds are described in WO 97/03642, GB 2286774, EP 743309, WO 98/22447 and GB 2319523.

Among the UV-screening agents of the triazine type in accordance with the invention, mention may also be made of insoluble s-triazine derivatives bearing benzalmalonate and/or phenylcyanoacrylate groups such as those described in patent application EP-A-0 790 243 (forming an integral part of the content of the description).

Among these insoluble UV-screening agents of the triazine type, mention will be made more particularly of the following compounds:

- 2,4,6-tris(diethyl 4'-aminobenzalmalonate)-s-triazine,

- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

Among the UV-screening agents of the triazine type in accordance with the invention, mention may also be made of insoluble s-triazine derivatives bearing benzotriazole and/or benzothiazole groups such as those described in patent application WO 98/25922 (forming an integral part of the content of the description).

Among these compounds, mention may be made more particularly of:

- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-tert-octyl)phenylamino]-s-triazine.

Mention may also be made of the symmetrical triazines substituted with naphthalenyl groups or polyphenyl groups described in patent US 6 225 467, patent application WO 2004/085 412 (see compounds 6 and 9) or the document "Symmetrical Triazine Derivatives", IP.COM Journal, IP.COM INC, West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985.

C/ Benzotriazoles

Among the insoluble organic UV-screening agents of the benzotriazole type in accordance with the invention, mention may be made of those of formula (III) below as described in patent application WO 95/22959 (forming an integral part of the content of the description):

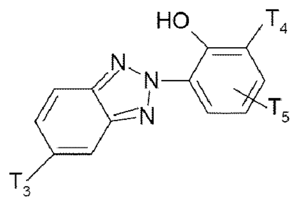

(III)

in which $T_7$ denotes a hydrogen atom or a $C_1$-$C_{18}$ alkyl radical; $T_8$ and $T_9$, which may be identical or different, denote a $C_1$-$C_{18}$ alkyl radical optionally substituted with a phenyl.

As examples of compounds of formula (III), mention may be made of the commercial products Tinuvin 328, 320, 234 and 350 from the company BASF, having the following structure:

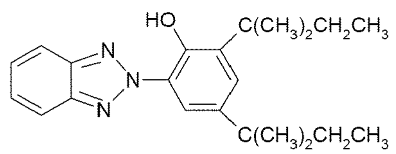

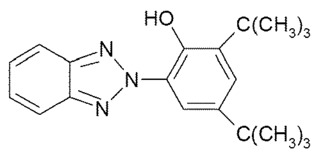

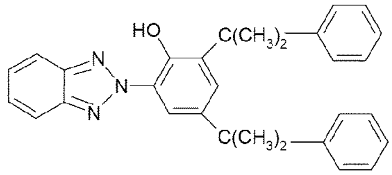

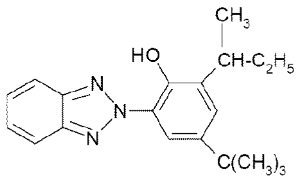

Among the insoluble organic UV-screening agents of the benzotriazole type in accordance with the invention, mention may be made of the compounds as described in patents US 5 687 521, US5 373 037 and US 5 362 881 and in particular [2,4'-dihydroxy-3-(2H-benzotriazol-2-yl)-5-(1,1,3,3-tetramethylbutyl)-2'-n-octoxy-5'-benzoyl]diphenylmethane sold under the name Mixxim PB30® by the company Fairmount Chemical, having the structure:

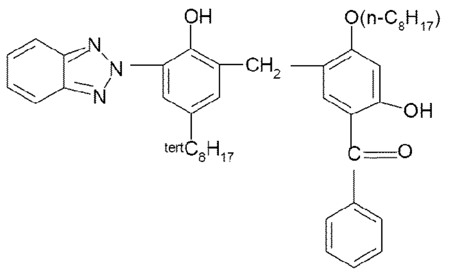

Among the insoluble organic UV-screening agents of the benzotriazole type in accordance with the invention, mention may be made of the methylenebis(hydroxyphenylbenzotriazole) derivatives having the following structure:

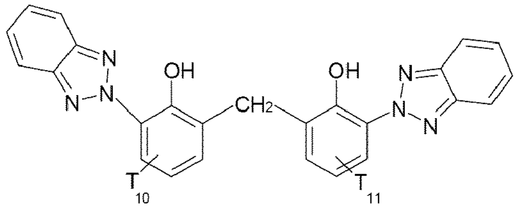

(IV)

in which the radicals $T_{10}$ and $T_{11}$, which may be identical or different, denote a $C_1$-$C_{18}$ alkyl radical possibly substituted with one or more radicals chosen from $C_1$-$C_4$ alkyl and $C_5$-$C_{12}$ cycloalkyl or an aryl residue. These compounds are known per se and are described in patent applications US 5237 071, US 5 166 355, GB-A-2 303 549, DE 197 26 184 and EP-A-893 119 (forming an integral part of the description).

In formula (I) defined above, the $C_1$-$C_{18}$ alkyl groups may be linear or branched and are, for example, methyl, ethyl, n-propyl, isopropyl, n-butyl, isobutyl, tert-butyl, tert-octyl, n-amyl, n-hexyl, n-heptyl, n-octyl, isooctyl, n-nonyl, n-decyl, n-undecyl, n-dodecyl, tetradecyl, hexydecyl or octadecyl; the $C_5$-$C_{12}$ cycloalkyl groups are, for example, cyclopentyl, cyclohexyl or cyclooctyl; the aryl groups are, for example, phenyl or benzyl.

Among the compounds of formula (IV), mention may be made of those having the following structure:

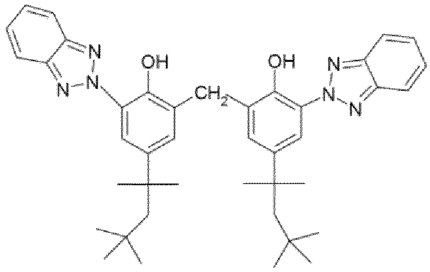

compound (a)

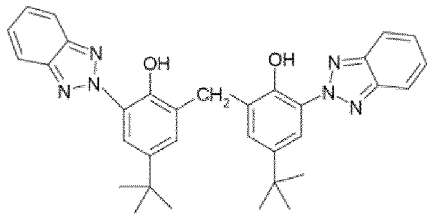

compound (b)

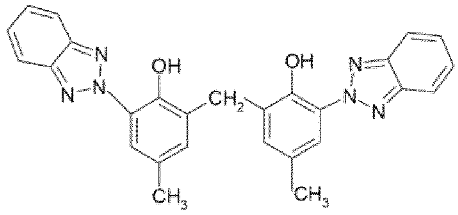

compound (c)

Compound (a) with the nomenclature 2,2'-methylenebis[6-(2H-benzotriazol-2-yl)-4-(1,1,3,3-tetramethylbutyl)phenol] is especially sold under the trade name Mixxim BB/200® by the company Fairmount Chemical.

Compound (c) with the nomenclature 2,2'-methylenebis[6-(2H-benzotriazol-2-yl)-4-(methyl)phenol] is especially sold in solid form under the trade name Mixxim BB/200® by the company Fairmount Chemical.

D/ Vinylamides

Among the insoluble organic screening agents of the vinylamide type, mention may be made, for example, of the compounds having the following formulae, which are described in application WO 95/22959 (forming an integral part of the content of the description):

$$T_{12}\text{-}(Y)r\text{-}C(=O)\text{-}C(T_{13})=C(T_{14})\text{-}N(T_{15})(T_{16}) \qquad (V)$$

in which $T_{12}$ is a $C_1$-$C_{18}$, preferably $C_1$-$C_5$, alkyl radical or a phenyl group optionally substituted with one, two or three radicals chosen from OH, $C_1$-$C_{18}$ alkyl, $C_1$-$C_8$ alkoxy or a group -C(=O)-O$T_{17}$ where $T_{17}$ is a $C_1$-$C_{18}$ alkyl group; $T_{13}$, $T_{14}$, $T_{15}$ and $T_{16}$, which may be identical or different, denote a $C_1$-$C_{18}$ and preferably $C_1$-$C_5$ alkyl radical, or a hydrogen atom; Y is N or O and r is 0 or 1.

Among these compounds, mention will more particularly be made of:

- 4-octylamino-3-penten-2-one;
- ethyl 3-octylamino-2-butenoate;
- 3-octylamino-1-phenyl-2-buten-1-one;
- 3-dodecylamino-1-phenyl-2-buten-1-one.

E/ Cinnamides

Among the insoluble organic screening agents of the cinnamide type in accordance with the invention, mention may also be made of the compounds as described in patent application WO 95/22959 (forming an integral part of the content of the description) and corresponding to the following structure:

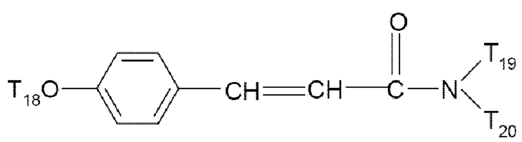

(VI)

in which O$T_{18}$ is a hydroxyl or $C_1$-$C_4$ alkoxy radical, preferably methoxy or ethoxy; $T_{19}$ is hydrogen, $C_1$-$C_4$ alkyl, preferably methyl or ethyl; $T_{20}$ is a group -(CONH)$_s$-phenyl in which s is 0 or 1 and the phenyl group may be substituted with one, two or three groups chosen from OH, $C_1$-$C_{18}$ alkyl, $C_1$-$C_8$ alkoxy or a group -C(=O)-O$T_{21}$ in which $T_{21}$ is a $C_1$-$C_{18}$ alkyl and more preferentially $T_{21}$ is a phenyl, 4-methoxyphenyl or phenylaminocarbonyl group.

Mention may also be made of cinnamide dimers such as those described in patent US 5 888 481, for instance the compound of structure:

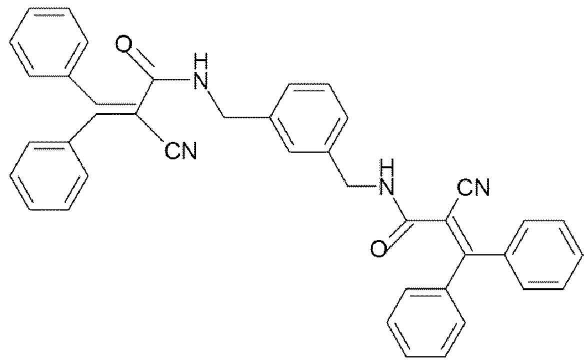

F/ Benzazoles

Among the insoluble organic screening agents of the benzazole type, mention may be made of those corresponding to one of the following formulae:

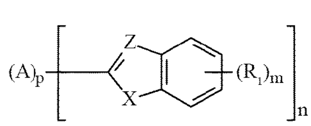

(VII)

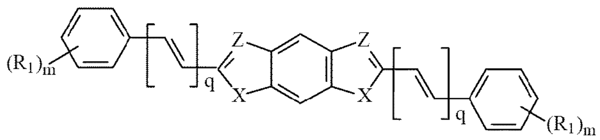

(VIII)

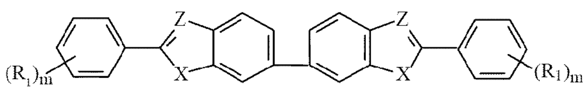

(IX)

in which each of the symbols X independently represents an oxygen or sulfur atom or a group $NR_2$, each of the symbols Z independently represents a nitrogen atom or a CH group,
each of the symbols $R_1$ independently represents an OH group, a halogen atom, a linear or branched $C_1$-$C_8$alkyl group optionally containing a silicon atom, or a linear or branched $C_1$-$C_8$alkoxy group,
each of the numbers m is independently 0, 1 or 2,
n represents an integer between 1 and 4, inclusive,
p is equal to 0 or 1,
each of the numbers q is independently equal to 0 or 1,
each of the symbols R2 independently represents a hydrogen atom, a benzyl group or a linear or branched $C_1$-$C_8$ alkyl group optionally containing a silicon atom,
A represents a radical of valency n chosen from those having the formulae:

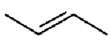

(a)

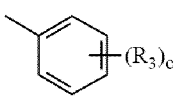

(b)

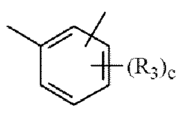

(c)

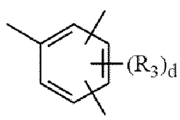

(d)

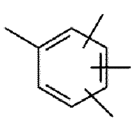

(e)

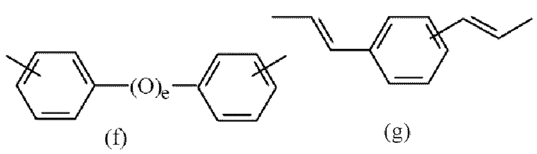

(f) (g)

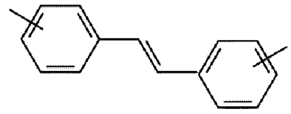

(h)

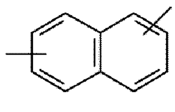

(i)

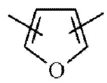

(j)

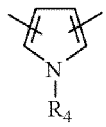

(k)

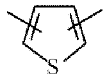

(l)

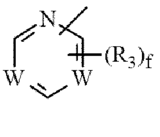

(m)

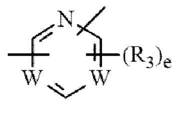

(n)

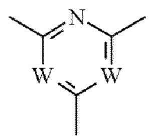

(o)

in which each of the symbols $R_3$ independently represents a halogen atom or a linear or branched $C_{1-4}$ alkyl or alkoxy, or hydroxyl, group, $R_4$ represents a hydrogen atom or a linear or branched $C_{1-4}$ alkyl group, c = 0 - 4, d = 0 - 3, e = 0 or 1, and f = 0 - 2.

These compounds are in particular described in patents DE 676 103 and CH 350 763, patent US 5 501 850, patent US 5 961 960, patent application EP 0 669 323, patent US 5 518 713, patent US 2 463 264, the article in J. Am. Chem. Soc., 79, 5706 - 5708, 1957, the article in J. Am. Chem. Soc., 82, 609 - 5,611, 1960, patent application EP 0 921 126 and patent application EP 0712855.

As examples of preferred compounds of formula (VII) of the 2-arylbenzazole family, mention may be made of 2-benzoxazol-2-yl-4-methylphenol, 2-(1H-benzimidazol-2-yl)-4-methoxyphenol or 2-benzothiazol-2-ylphenol, it being possible for these compounds to be prepared, for example, according to the processes described in patent CH 350 763.

As examples of preferred compounds of formula (VII) of the benzimidazolylbenzazole family, mention will be made of 2,2'-bis-benzimidazole, 5,5',6,6'-tetramethyl-2,2'-bisbenzimidazole, 5,5'-dimethyl-2,2'-bisbenzimidazole, 6-methoxy-2,2'-bisbenzimidazole, 2-(1H-benzimidazol-2-yl)benzothiazole, 2-(1H-benzimidazol-2-yl)benzoxazole and N,N'-dimethyl-2,2'-bisbenzimidazole, it being possible for these compounds to be prepared according to the procedures described in patents US 5 961 960 and US 2 463 264.

As examples of preferred compounds of formula (VII) of the phenylenebenzazole family, mention will be made of 1,4-phenylenebis(2-benzoxazole), 1,4-phenylenebis(2-benzimidazole), 1,3-phenylenebis(2-benzoxazole), 1,2-phenylenebis(2-benzoxazole), 1,2-phenylenebis(benzimidazole), 1,4-phenylenebis(N-2-ethylhexyl-2-benzimidazole) and 1,4-phenylenebis(N-trimethylsilylmethyl-2-benzimidazole), it being possible for these compounds to be prepared according to the procedures described in patent US 2 463 264 and in the publications J. Am. Chem. Soc., 82, 609 (1960) and J. Am. Chem. Soc., 79, 5706 - 5708 (1957).

As examples of preferred compounds of formula (VII) of the benzofuranylbenzoxazole family, mention will be made of 2-(2-benzofuranyl)benzoxazole, 2-(benzofuranyl)-5-methylbenzoxazole and 2-(3-methyl-2-benzofuranyl)benzoxazole, it being possible for these compounds to be prepared according to the procedures described in patent US 5 518 713.

As preferred compounds of formula (VIII), mention may be made, for example, of 2,6-diphenyl-1,7-dihydrobenzo[1,2-d;4,5-d']diimidazole corresponding to the formula:

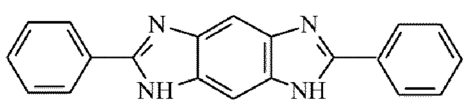

or 2,6-distyryl-1,7-dihydrobenzo[1,2-d; 4,5-d']diimidazole or else 2,6-di(p-tert-butylstyryl)-1,7-dihydrobenzo[1,2-d; 4,5-d']diimidazole, which can be prepared according to the processes described in application EP 0 669 323.

As preferred compound of formula (IX), mention may be made of 5,5'-bis-[(phenyl-2)benzimidazole] having the

formula:

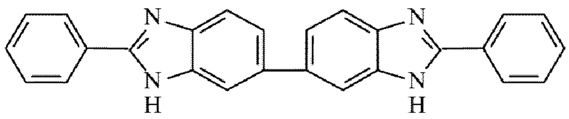

the preparation of which is described in J. Chim. Phys., 64, 1602 (1967).

Among these UV radiation-screening organic insoluble compounds, 2-(1H-benzimidazol-2-yl)benzoxazole, 5 ole, 6-methoxy-2,2'-bisbenzimidazole, 2-(1H-benzimidazol-2-yl)benzothiazole, 1,4-phenylenebis(2-benzoxazole), 1,4-phenylenebis(2-benzimidazole), 1,3-phenylenebis(2-benzoxazole), 1,2-phenylenebis(2-benzoxazole), 1,2-phenylenebis(2-benzimidazole) and 1,4-phenylenebis(N-trimethylsilylmethyl-2-benzimidazole) are most particularly preferred.

<u>G/ Aryl vinylene ketones</u>

Among the insoluble organic screening agents of the aryl vinylene ketone type, mention may be made of those corresponding to one of the following formulae (X) and (XI):

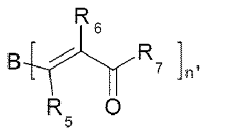

(X)

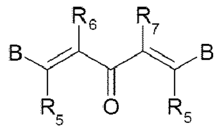

(XI)

in which:

n' = 1 or 2,

B, in formula (X) when n' = 1 or in formula (XI), is an aryl radical chosen from the following formulae (a') to (d') or in formula (X) when n' = 2, is a radical chosen from the following formulae (e') to (h'):

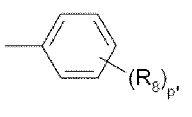
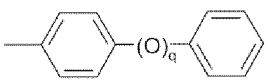
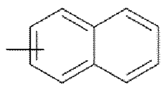
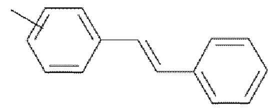

(a') (b') (c') (d')

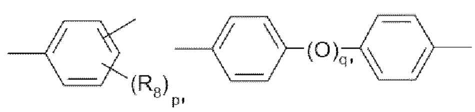
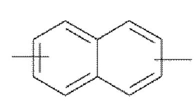
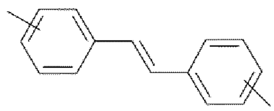

(e') (f') (g') (h')

in which:

each of the symbols $R_8$ independently represents an OH group, a halogen atom, a linear or branched $C_1$-$C_6$ alkyl group optionally containing a silicon atom, a linear or branched $C_1$-$C_6$ alkoxy group optionally containing a silicon atom, a linear or branched $C_1$-$C_5$ alkoxycarbonyl group, or a linear or branched $C_1$-$C_6$ alkylsulfonamide group optionally containing a silicon atom or an amino acid function,

p' represents an integer between 0 and 4 inclusive,

q' represents 0 or 1,

$R_5$ represents hydrogen or an OH group,
$R_6$ represents hydrogen, a linear or branched $C_1$-$C_6$ alkyl group optionally containing a silicon atom, a cyano group, a $C_1$-$C_6$ alkylsulfonyl group or a phenylsulfonyl group,
$R_7$ represents a linear or branched $C_1$-$C_6$ alkyl group optionally containing a silicon atom or a phenyl group which can form a bicycle and which is optionally substituted with one or two $R_4$ radicals,
or $R_6$ and $R_7$ together form a $C_{2-10}$ monocyclic, bicyclic or tricyclic hydrocarbon-based residue, optionally interrupted with one or more nitrogen, sulfur and oxygen atoms, and possibly containing another carbonyl, and optionally substituted with a linear or branched $C_1$-$C_8$ alkylsulfonamide group,
and optionally containing a silicon atom or an amino acid function; on condition that when n' = 1, $R_6$ and $R_7$ do not form a camphor nucleus.

As examples of insoluble UV radiation-screening compounds of formula (X) in which n' = 1, with a mean particle size of between 10 nm and 5 nm, mention may be made of the following families:

- compounds of the styryl ketone type as described in patent application JP 04 134 042, such as 1-(3,4-dimethoxyphenyl)-4,4-dimethylpent-1-en-3-one:

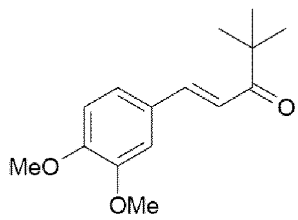

- compounds of the benzylidene cineole type such as those described in the article by E. Mariani et al., 16th IFSCC Congress, New York (1990), such as 1,3,3-trimethyl-5-(4-methoxybenzylidene)-2-oxabicyclo[2.2.2]octan-6-one:

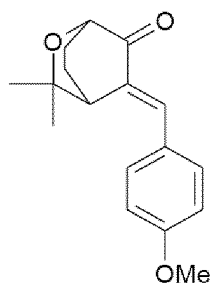

- compounds of the benzylidene chromanone type such as those described in patent application JP 04 134 043, for instance 3-(4-methoxybenzylidene)-2,3,4a,8a-tetrahydrochromen-4-one:

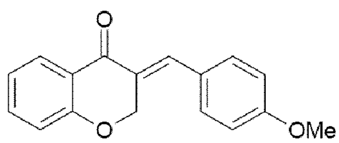

- compounds of the benzylidene thiochromanone type such as those described in patent application JP 04 134 043, for instance 3-(4-methoxybenzylidene)-2,3,4a,8a-tetrahydrochromene-4-thione:

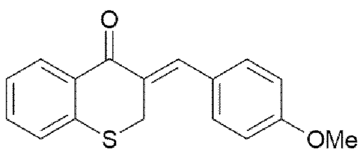

- compounds of the benzylidene quinuclidinone type such as those described in patent application EP 0 576 974, for instance 4-methoxybenzyliden--1-azabicyclo[2.2.2]octan-3-one:

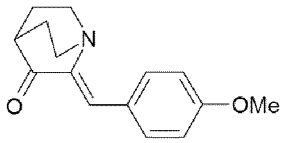

- compounds of the benzylidene cycloalkanone type such as those described in patent application FR 2 395 023, for instance 2-(4-methoxybenzylidene)cyclopentanone and 2-(4-methoxybenzylidene)cyclohexanone:

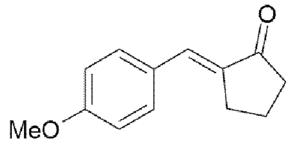

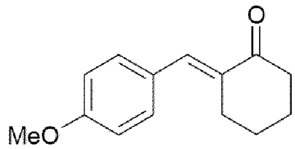

- compounds of the benzylidene hydantoin type such as those described in patent application JP 01 158 090, for instance 5-(3,4-dimethoxybenzylidene)imidazolidine-2,4-dione:

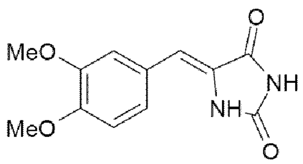

- compounds of the benzylidene hydantoin type such as those described in patent application JP 04 134 043, for instance 2-(4-methoxybenzylidene)indan-1-one:

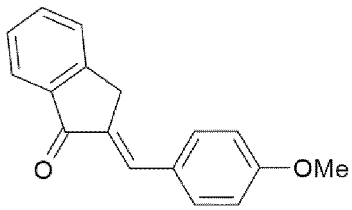

- compounds of the benzylidene tetralone type such as those described in patent application JP 04 134 043, for instance 2-(4-methoxybenzylidene)-3,4-dihydro-2H-naphthalen-1-one:

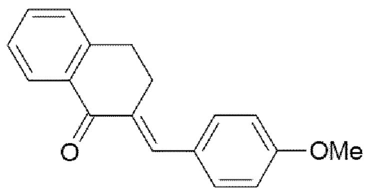

- compounds of the benzylidene furanone type such as those described in patent application EP 0 390 683, for instance 4-(4-methoxybenzylidene)-2,2,5,5-tetramethyldihydrofuran-3-one:

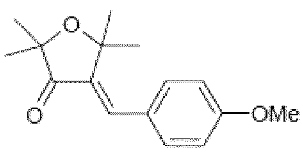

- compounds of the benzylidene benzofuranone type such as those described in patent application JP 04 134 041, for instance 2-benzylidenebenzofuran-3-one:

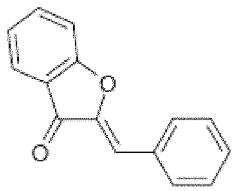

- compounds of the benzylidene indanedione type such as 2-(3,5-di-tert-butyl-4-hydroxybenzylidene)indane-1,3-dione:

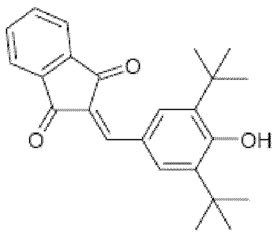

- compounds of the benzylidene benzothiofuranone type such as those described in patent application JP 04 134 043, for instance 2-benzylidenebenzo[b]thiophen-3-one:

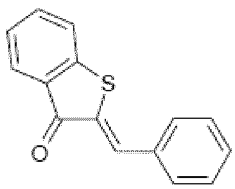

- compounds of the benzylidene barbituric type such as 5-(4-methoxybenzylidene)-1,3-dimethylpyrimidine-2,4,6-trione:

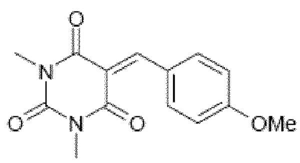

- compounds of the benzylidene pyrazolone type such as 4-(4-methoxybenzylidene)-5-methyl-2-phenyl-2,4-dihydropyrazol-3-one:

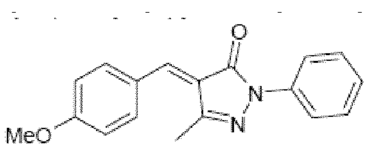

- compounds of the benzylidene imidazolone type such as 5-(4-methoxybenzylidene)-2-phenyl-3,5-dihydroimidazol-4-one:

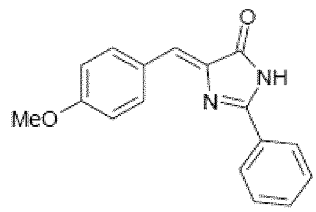

- compounds of the chalcone type such as 1-(2-hydroxy-4-methoxyphenyl)-3-phenylpropenone:

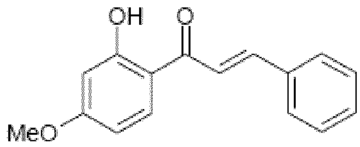

- the benzylidene one compounds as described in document FR 2 506 156 for instance 3-hydroxy-1-(2-hydroxy-4-methoxyphenyl)-3-phenylpropenone:

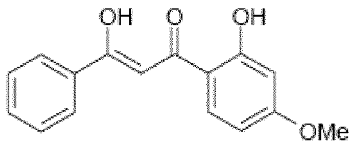

As examples of insoluble UV radiation-screening compounds of formula (X) in which n' = 2, with a mean particle size of between 10 nm and 5 μm, mention may be made of the following families:

- compounds of the phenylene bis methylidene-nor-camphor type such as those described in document EP 0 693 471, for instance 1,4-phenylenebis{3-methylidenebicyclo[2.2.1]heptan-2-one}:

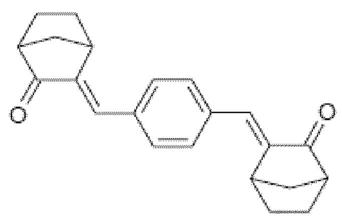

- compounds of the phenylene bis methylidene camphor type such as those described in document FR 2 528 420, for instance 1,4-phenylenebis{3-methylidene-1,7,7-trimethylbicyclo[2.2.1]heptan-2-one}:

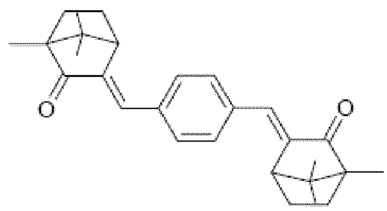

or 1,3-phenylenebis{3-methylidene-1,7,7-trimethylbicyclo[2.2.1]heptan-2-one}:

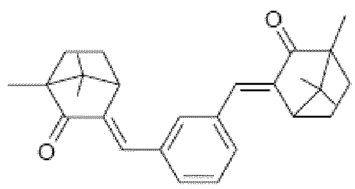

- compounds of the phenylene bis methylidene camphor sulfonamide type such as those described in document FR2 529 887, for instance ethyl or 2-ethylhexyl 1,4-phenylenebis(3,3'-methylidenecamphor-10,10'-sulfonamide):

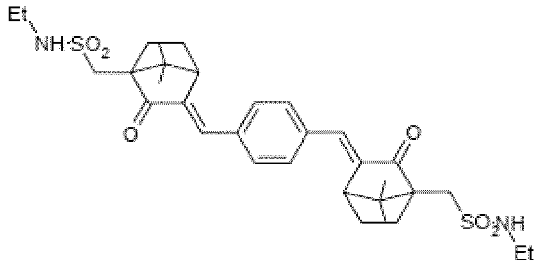

or

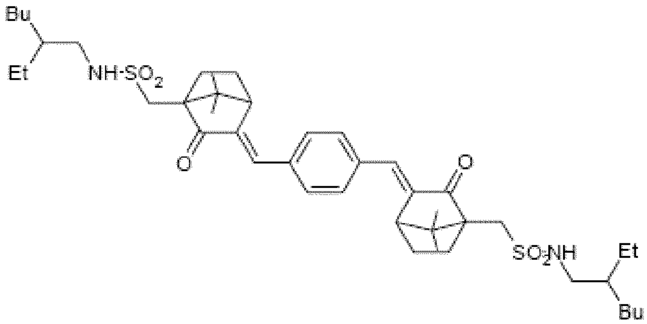

- compounds of the phenylene bis methylidene cineole type such as those described in the article by E. Mariani et al., 16th IFSCC Congress, New York (1990), such as 1,4-phenylenebis{5-methylidene-3,3-dimethyl-2-oxabicyclo[2.2.2]octan-6-one}:

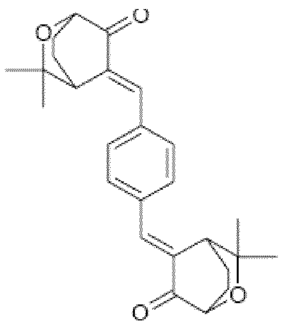

- compounds of the phenylene bis methylidene ketotricyclodecane type such as those described in patent application EP 0 694 521, for instance 1,4-phenylenebis(octahydro-4,7-methano-6-inden-5-one):

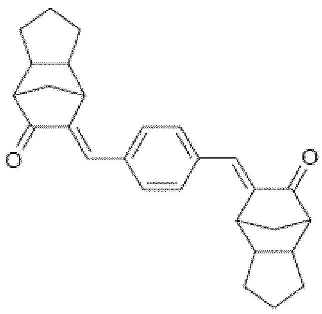

- compounds of the phenylene bis alkylene ketone type such as those described in patent application JP 04 134 041,

for instance 1,4-phenylenebis(4,4-dimethylpent-1-en-3-one):

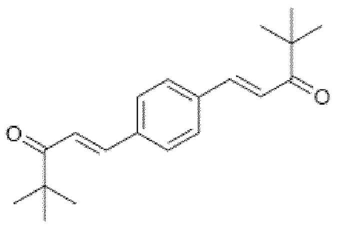

- compounds of the phenylene bis methylidene furanone type such as those described in patent application FR 2 638 354, for instance 1,4-phenylenebis(4-methylidene-2,2,5,5-tetramethyldihydrofuran-3-one):

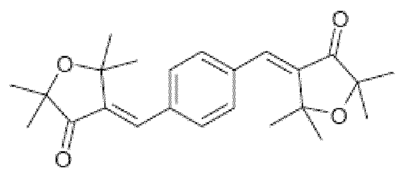

- compounds of the phenylene bis methylidene quinuclidinone type such as those described in patent application EP 0 714 880, for instance 1,4-phenylenebis{2-methylidene-1-aza-bicyclo[2.2.2]octan-3-one}:

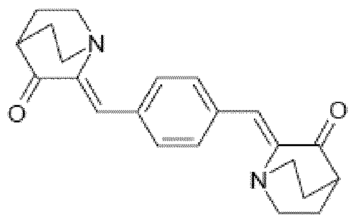

As compounds of formula (XI), mention may be made of the following families:

- compounds of the bis benzylidene cycloalkanone type such as 2,5-dibenzylidenecyclopentanone:

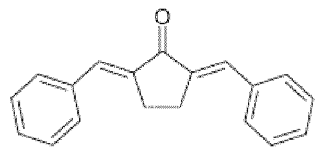

- compounds of the gamma pyrone type such as those described in document JP 04 290 882, for instance 2,6-bis(3,4-dimethoxyphenyl)pyran-4-one:

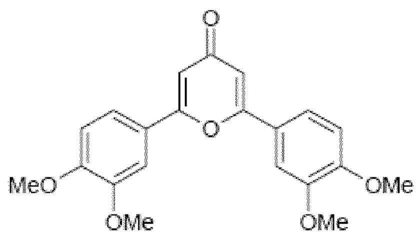

Among these insoluble organic UV radiation-screening compounds of the aryl vinylene ketone type, the ones most particularly preferred are the compounds of formula (X) in which n' = 2.

H/ Phenylene bis-benzoxazinones

Among the insoluble organic screening agents of the phenylene bis-benzoxazinone type, mention may be made of those corresponding to formula (XII) below:

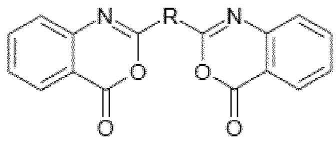

(XII)

with R representing a divalent aromatic residue chosen from the following formulae (e) to (h):

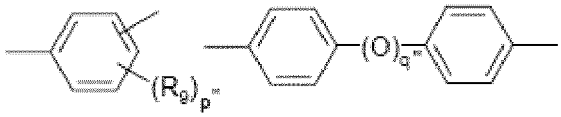

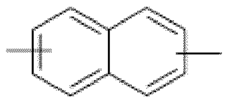

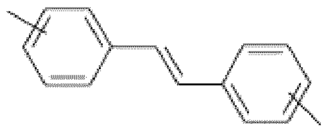

(e") (f") (g") (h")

in which:

each of the symbols $R_9$ independently represents an OH group, a halogen atom, a linear or branched $C_1$-$C_6$ alkyl group optionally containing a silicon atom, a linear or branched $C_1$-$C_6$ alkoxy group optionally containing a silicon atom, a linear or branched $C_1$-$C_5$ alkoxycarbonyl group, or a linear or branched $C_1$-$C_6$ alkylsulfonamide group optionally containing a silicon atom or an amino acid function,
p" represents an integer between 0 and 4, inclusive,
q" represents 0 or 1.

As examples of insoluble UV radiation-screening compounds of formula (XII), with a mean particle size of between 10 nm and 5 μm, mention may be made of the following derivatives:

- 2,2'-p-phenylenebis(3,1-benzoxazin-4-one), the commercial product Cyasorb UV-3638® from the company Cytec,
- 2,2'-(4,4'-biphenylene)bis(3,1-benzoxazin-4-one),
- 2,2'-(2,6-naphthylene)bis(3,1-benzoxazin-4-one).

I/ Acrylonitrile amide, sulfonamide or carbamate derivatives

Among the insoluble organic screening agents of the acrylonitrile amide, sulfonamide or carbamate type, mention may be made of those corresponding to formula (XIII) below:

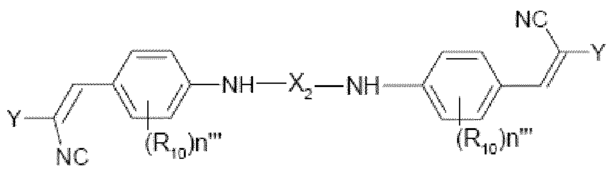

(XIII)

in which:

$R_{10}$ represents a linear or branched $C_1$-$C_8$ alkyl group,
n''' is 0, 1 or 2,
$X_2$ represents a divalent radical of formula -(C=O)-$R_{11}$-(C=O)-, -$SO_2$-$R_{11}$-$SO_2$- or-(C=O)-O-$R_{11}$-O-(C=O)-,
Y represents a radical -(C=O)-$R_{12}$ or -$SO_2R_{13}$,
$R_{11}$ represents a single bond or a linear or branched divalent $C_1$-$C_{30}$ alkylene or $C_3$-$C_{30}$ alkenylene radical, which may bear one or more hydroxyl substituents and which may contain in the carbon chain one or more heteroatoms chosen from oxygen, nitrogen and silicon atoms,

$R_{12}$ represents a radical $-OR_{14}$ or $-NHR_{14}$,
$R_{13}$ represents a linear or branched $C_1$-$C_{30}$ alkyl radical, or a phenyl nucleus which is unsubstituted or substituted with $C_1$-$C_4$ alkyl or alkoxy radicals,
$R_{14}$ represents a linear or branched $C_1$-$C_{30}$ alkyl or $C_3$-$C_{30}$ alkenyl radical, which may bear one or more hydroxyl substituents and which may contain in the carbon-based chain one or more heteroatoms chosen from oxygen, nitrogen and silicon atoms.

Although in formula (XIII) above only the isomers in which the cyano substituent is in the *cis* position relative to the para-aminophenyl substituent are represented, this formula should be understood as also encompassing the corresponding *trans* isomers; for each of the two double bonds, and independently, the cyano and para-aminophenyl substituents may be in the *cis* or *trans* configuration relative to each other.
An example that may be mentioned is the 2-ethylhexyl 2-cyano-3-[4-(acetylamino)phenyl]acrylate dimer of formula:

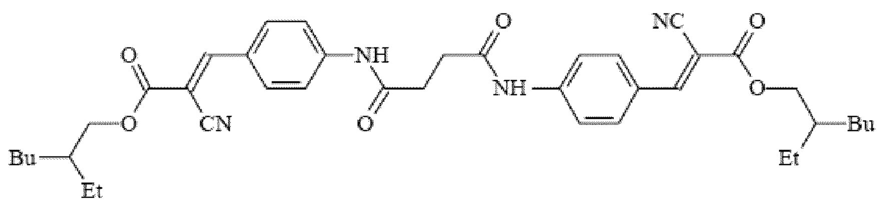

J/ Polyvalent metals

Another particular family of insoluble organic screening agents in accordance with the invention is that of polyvalent metal salts (for example of $Ca^{2+}$, $Zn^{2+}$, $Mg^{2+}$, $Ba^{2+}$, $Al^{3+}$ or $Zr^{4+}$) of sulfonic or carboxylic organic screening agents, such as the polyvalent metal salts of sulfonic derivatives of benzylidenecamphor, such as those described in patent application FR-A 2 639 347; the polyvalent metal salts of sulfonic derivatives of benzimidazole such as those described in patent application EP-A-893 119; the polyvalent metal salts of cinnamic acid derivatives, such as those described in application JP-87 166 517.
Mention may also be made of metal or ammonium or substituted ammonium complexes of UV-A and/or UV-B organic screening agents, such as those described in patent applications WO 93/10753, WO 93/11095 and WO 95/05150.
Among the insoluble organic UV-screening agents, mention may also be made of compound 1,1'-(1,4-piperazinediyl)bis[1-[2-[4-(diethylamino)-2-hydroxybenzoyl]phenyl]methanone (CAS 919803-06-8) having the following structure:

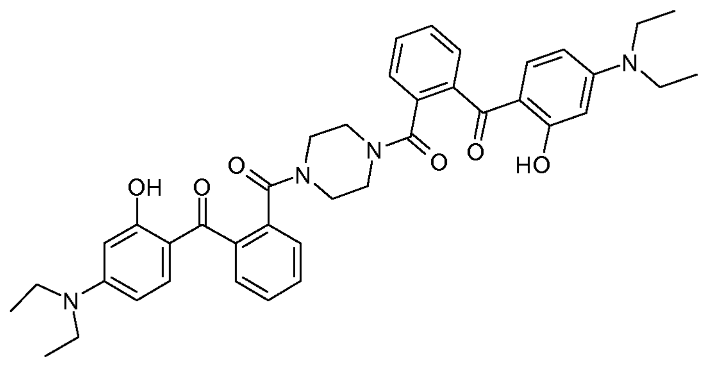

as described in patent application WO 2007/071584; this compound advantageously being used in micronized form (mean size of 0.02 to 2 μm), which may be obtained, for example, according to the micronization process described in patent applications GB-A-2 303 549 and EP-A-893 119, and in particular in the form of an aqueous dispersion. According to a particularly preferred form of the invention, use will be made of insoluble organic UV-screening agents chosen from

(i) symmetrical triazine screening agents substituted by naphthalenyl groups or polyphenyl groups described in patent US 6 225 467, patent application WO 2004/085412 (see compounds 6 and 9) or the document "Symmetrical Triazine Derivatives", IP.COM IPCOM000031257 Journal, INC, West Henrietta, NY, US (20 September 2004), in particular 2,4,6-tris(diphenyl)triazine and 2,4,6-tris(terphenyl)triazine, which is also mentioned in patent applications

WO 06/035000, WO 06/034982, WO 06/034991, WO 06/035007, WO 2006/034992 and WO 2006/034985, these compounds advantageously being used in the micronized form (mean particle size of 0.02 to 3 μm), which may be obtained, for example, according to the micronization process described in patent applications GB-A-2 303 549 and EP-A-893 119, and in particular in the form of an aqueous dispersion;

(ii) the methylenebis(hydroxyphenylbenzotriazole) compounds of formula (IV) below:

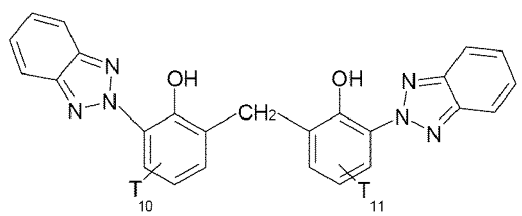

(IV)

in which the radicals $T_{10}$ and $T_{11}$, which may be identical or different, denote a $C_1$-$C_{18}$ alkyl radical possibly substituted with one or more radicals chosen from $C_1$-$C_4$ alkyl and $C_5$-$C_{12}$ cycloalkyl or an aryl residue;

(iii) and mixtures thereof.

According to a particularly preferred form of the invention, the methylenebis(hydroxyphenylbenzotriazole) compounds of formula (IV) are in the form of an aqueous dispersion of particles with a mean particle size which ranges from 0.01 to 5 μm, more preferentially from 0.01 to 2 μm and more particularly from 0.020 to 2 μm with at least one surfactant of structure $C_nH_{2n+1}\ O(C_6H_{10}O_5)_xH$ in which n is an integer from 8 to 16 and x is the average degree of polymerization of the unit $(C_6H_{10}O_5)$ and ranges from 1.4 to 1.6 as defined previously. The said surfactant is preferably used at a concentration ranging from 1% to 50% by weight and more preferentially from 5% to 40% by weight relative to the benzotriazole screening agent and the amount of benzotriazole screening agent of formula (I) in the aqueous dispersion preferably ranges from 10% to 50% by weight and more preferentially from 30% to 50% by weight relative to the total weight of the dispersion.

The mean particle diameter is measured with a particle size distribution analyser such as the Coulter N4 Plus® machine manufactured by Beckman Coulter Inc.

According to a particularly preferred form of the invention, the methylenebis(hydroxyphenylbenzotriazole) compounds of formula (IV) may be in the form of an aqueous dispersion of particles with a mean particle size ranging from 0.02 to 2 μm, more preferentially from 0.01 to 1.5 μm and more particularly from 0.02 to 1 μm, in the presence of at least one polyglyceryl mono($C_8$-$C_{20}$)alkyl ester with a degree of glycerol polymerization of at least 5, such as the aqueous dispersions described in patent application WO 2009/063 392.

As examples of polyglyceryl mono($C_8$-$C_{20}$)alkyl ester surfactants, mention may be made of decaglyceryl caprate, decaglyceryl laurate, decaglyceryl myristate, decaglyceryl oleate, decaglyceryl stearate, decaglyceryl isostearate, hexaglyceryl caprate, hexaglyceryl laurate, hexaglyceryl myristate, hexaglyceryl oleate, hexaglyceryl stearate, hexaglyceryl isostearate, pentaglyceryl caprate, pentaglyceryl laurate, pentaglyceryl myristate, pentaglyceryl oleate, pentaglyceryl stearate and pentaglyceryl isostearate.

Use will be made in particular of:

- decaglyceryl caprate such as the products sold under the following trade names: Sunsoft Q10Y®, Sunsoft Q10S®, Sunsoft Q12Y®, Sunsoft Q12S®, Sunsoft M12J® by the company Taiyo Kagaku Co. Ltd., Nikkol Decaglyn 1-L by the company Nikko Chemicals Co. Ltd, Ryoto-Polyglyceryl ester L-10D® and L-7D® by the company Mitsubishi-Kagaku Co. Ltd.,
- decaglyceryl laurate such as the products sold under the following trade names: Sunsoft Q14Y®, Sunsoft Q14S®, Sunsoft Q12Y®, Sunsoft Q12S®, Sunsoft M12J® by the company Taiyo Kagaku Co. Ltd., Nikkol Decaglyn 1-M® by the company Nikko Chemicals Co. Ltd, Ryoto-Polyglyceryl ester M-10D® and M-7D® by the company Mitsubishi-Kagaku Co. Ltd.,
- decaglyceryl stearate such as the products sold under the following trade names: Sunsoft Q18Y®, Sunsoft Q18S®, Sunsoft Q12Y®, Sunsoft Q12S®, Sunsoft M12J® by the company Taiyo Kagaku Co. Ltd., Nikkol Decaglyn 1-SV by the company Nikko Chemicals Co. Ltd, Ryoto-Polyglyceryl ester S-15D® by the company Mitsubishi-Kagaku Co. Ltd.,
- hexaglyceryl caprate such as the products sold under the following trade names: Nikkol Hexaglyn 1-L® by the company Nikko Chemicals Co. Ltd, Glysurf 6ML by the company Aoki Oil Industrial Co. Ltd., Unigly GL-106® by the company Nippon Oil & Fats Co. Ltd.,
- hexaglyceryl myristate such as the products sold under the following trade names: Nikkol Hexaglyn 1-M®, Nikkol Hexaglyn 1-OV® by the company Nikko Chemicals Co. Ltd, Glysurf 6ML® by the company Aoki Oil Industrial Co.

Ltd., Unigly GL-106 by the company Nippon Oil & Fats Co. Ltd.,

- hexaglyceryl stearate such as the products sold under the following trade names: Nikkol Hexaglyn 1-M®, Nikkol Hexaglyn 1-SV® by the company Nikko Chemicals Co. Ltd, Emalex MSG-6K® by the company Nihon-Emulsion Co. Ltd., Unigly GL-106 by the company Nippon Oil & Fats Co. Ltd.,
- hexaglyceryl isostearate such as the product sold under the following trade name: Matsumate MI-610® by the company Matsumoto Fine Chemical Co. Ltd,
- pentaglyceryl caprate such as the product sold under the following trade name: Sunsoft A10E® by the company Taiyo Kagaku Co. Ltd.,
- pentaglyceryl laurate such as the products sold under the following trade names: Sunsoft A12E®, Sunsoft A121E® by the company Taiyo Kagaku Co. Ltd.,
- pentaglyceryl myristate such as the products sold under the following trade names: Sunsoft A14E®, Sunsoft A141E® by the company Taiyo Kagaku Co. Ltd.,
- pentaglyceryl oleate such as the products sold under the following trade names: Sunsoft A17E®, Sunsoft A171E® by the company Taiyo Kagaku Co. Ltd.,
- pentaglyceryl stearate such as the products sold under the following trade names: Sunsoft A18E®, Sunsoft A181E® by the company Taiyo Kagaku Co. Ltd.

Among these surfactants, use is preferably made of those with an HLB of greater than or equal to 14.5 and more preferentially greater than or equal to 15. As examples of polyglyceryl mono($C_8$-$C_{20}$)alkyl ester surfactants with a degree of polymerization of glycerol of at least 5 and and with an HLB of greater than or equal to 14.5, mention may be made of decaglyceryl caprate, decaglyceryl laurate, decaglyceryl myristate, decaglyceryl oleate, decaglyceryl stearate, decaglyceryl isostearate, hexaglyceryl laurate, pentaglyceryl caprate, pentaglyceryl laurate, pentaglyceryl myristate, pentaglyceryl oleate and pentaglyceryl stearate. As examples of polyglyceryl mono($C_8$-$C_{20}$)alkyl ester surfactants with a degree of polymerization of glycerol of at least 5 and and with an HLB of greater than or equal to 15, mention may be made of decaglyceryl caprate and decaglyceryl laurate.

The amount of methylenebis(hydroxyphenylbenzotriazole) compound of formula (IV) in the aqueous dispersion preferably ranges from 10% to 50% by weight and more preferentially from 30% to 50% by weight relative to the total weight of the dispersion.

Preferentially, the weight ratio of methylenebis(hydroxyphenylbenzotriazole) compound/polyglyceryl mono($C_8$-$C_{20}$)alkyl ester ranges from 0.05 to 0.5 and more preferentially from 0.1 to 0.3.

Use will be made more preferentially in these aqueous dispersions, as methylenebis(hydroxyphenylbenzotriazole) compound of formula (IV), of the compound 2,2'-methylenebis[6-(2H-benzotriazol-2-yl)-4-(1,1,3,3-tetramethylbutyl)phenol] of structure:

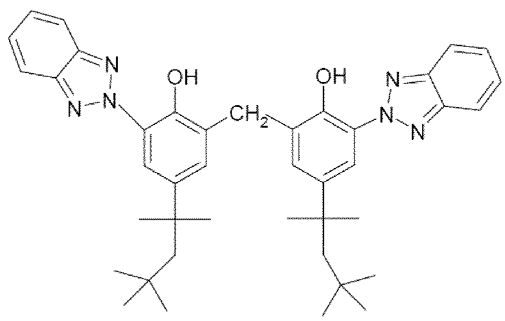

compound (a)

such as the commercial product sold under the name Tinosorb M® by BASF, which is an aqueous dispersion comprising decylglucoside, xanthan gum and propylene glycol (INCI name: Methylene Bis-Benzotriazolyl Tetramethylbutylphenol (and) Aqua (and) Decyl Glucoside (and) Propylene Glycol (and) Xanthan Gum).

The insoluble organic UV-screening agent(s) of the invention are present in an active material concentration preferably ranging from about 0.1% to 15% by weight and more particularly from 0.5% to 10% by weight relative to the total weight of the composition.

## INSOLUBLE INORGANIC UV-SCREENING AGENTS

The inorganic UV-screening agents used in accordance with the present invention are metal oxide pigments. More preferentially, the inorganic UV-screening agents of the invention are metal oxide particles with a mean elementary particle size of less than or equal to 0.5 μm, more preferentially between 0.005 and 0.5 μm, even more preferentially

between 0.01 and 0.2 μm, better still between 0.01 and 0.1 μm and more particularly preferentially between 0.015 and 0.05 μm.

They may be selected in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara and UVT 14/4 from the company Sachtleben Pigments,
- with alumina and aluminium stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminium laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR 351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminium stearate and treated with a silicone, such as the product STT-30-DS from the company Titan Kogyo,
- with silica and treated with a silicone, such as the product UV-Titan X 195 from the company Sachtleben Pigments,
- with alumina and treated with a silicone, such as the products Tipaque TTO-55 (S) from the company Ishihara or UV Titan M 262 from the company Sachtleben Pigments,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca.
- $TiO_2$ treated with octyltrimethylsilane, sold under the trade name T 805 by the company Degussa Silices,
- $TiO_2$ treated with a polydimethylsiloxane, sold under the trade name 70250 Cardre UF TiO2SI3 by the company Cardre,
- anatase/rutile $TiO_2$ treated with a polydimethylhydrosiloxane, sold under the trade name Microtitanium Dioxide USP Grade Hydrophobic by the company Color Techniques.

Mention may also be made of $TiO_2$ pigments doped with at least one transition metal such as iron, zinc or manganese and more particularly manganese. Preferably, the said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50 by the company Croda.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackherr under the name Transparent titanium oxide PW, by the company Miyoshi Kasei under the

name UFTR, by the company Tomen under the name ITS and by the company Tioxide under the name Tioveil AQ.

The uncoated zinc oxide pigments are for example:

- those sold under the name Z-Cote by the company Sunsmart;
- those sold under the name Nanox by the company Elementis;
- those sold under the name Nanogard WCD 2025 by the company Nanophase Technologies.

The coated zinc oxide pigments are for example:

- those sold under the name Zinc Oxide CS-5 by the company Toshibi (ZnO coated with polymethylhydrogenosiloxane);
- those sold under the name Nanogard Zinc Oxide FN by the company Nanophase Technologies (as a 40% dispersion in Finsolv TN, C12-C15 alkyl benzoate);
- those sold under the name Daitopersion ZN-30 and Daitopersion ZN-50 by the company Daito (dispersions in cyclopolymethylsiloxane/oxyethylenated polydimethylsiloxane, containing 30% or 50% of zinc oxides coated with silica and polymethylhydrogenosiloxane);

- those sold under the name NFD Ultrafine ZnO by the company Daikin (ZnO coated with perfluoroalkyl phosphate and copolymer based on perfluoroalkylethyl as a dispersion in cyclopentasiloxane);
- those sold under the name SPD-Z1 by the company Shin-Etsu (ZnO coated with silicone-grafted acrylic polymer, dispersed in cyclodimethylsiloxane);
- those sold under the name Escalol Z100 by the company ISP (alumina-treated ZnO dispersed in an ethylhexyl methoxycinnamate/PVP-hexadecene copolymer/methicone mixture);
- those sold under the name Fuji ZnO-SMS-10 by the company Fuji Pigment (ZnO coated with silica and polymethylsilsesquioxane);
- those sold under the name Nanox Gel TN by the company Elementis (ZnO dispersed at a concentration of 55% in C12-C15 alkyl benzoate with hydroxystearic acid polycondensate).

The uncoated cerium oxide pigments may be, for example, those sold under the name Colloidal Cerium Oxide by the company Rhone-Poulenc.
The non-coated iron oxide pigments are sold, for example, by Arnaud under the names Nanogard WCD 2002 (FE 45B), Nanogard Iron FE 45 BL AQ, Nanogard FE 45R AQ and Nanogard WCD 2006 (FE 45R) or by Mitsubishi under the name TY-220.

The coated iron oxide pigments are sold, for example, by Arnaud under the names Nanogard WCD 2008 (FE 45B FN), Nanogard WCD 2009 (FE 45B 556), Nanogard FE 45 BL 345 and Nanogard FE 45 BL or by BASF under the name Transparent Iron Oxide.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Sachtleben Pigments, or coated with alumina, silica and glycerol, such as the product M 211 sold by the company Sachtleben Pigments.

According to the invention, coated or uncoated titanium oxide pigments are particularly preferred.

The insoluble inorganic UV-screening agents of the invention are preferably present in the compositions according to the invention in a content ranging from 0.1% to 30% by weight, more particularly from 0.5% to 20% by weight and in particular from 1% to 10% by weight relative to the total weight of the composition.

## OILY PHASE

The compositions in accordance with the invention comprise at least one oily phase.

For the purposes of the invention, the term "oily phase" is understood to mean a phase comprising at least one oil and all of the liposoluble and lipophilic ingredients and the fatty substances used for the formulation of the compositions of the invention.

The term "oil" is understood to mean any fatty substance in the liquid form at room temperature (20 - 25°C) and at atmospheric pressure (760 mmHg).

An oil suitable for the invention can be volatile or non-volatile.

An oil suitable for the invention can be chosen from hydrocarbon-based oils, silicone oils, fluorinated oils and mixtures thereof.

A hydrocarbon-based oil suitable for the invention can be an animal hydrocarbon-based oil, a vegetable hydrocarbon-based oil, a mineral hydrocarbon-based oil or a synthetic hydrocarbon-based oil.

An oil suitable for the invention can advantageously be chosen from mineral hydrocarbon-based oils, vegetable hydrocarbon-based oils, synthetic hydrocarbon-based oils, silicone oils and mixtures thereof.

For the purposes of the present invention, the term "silicone oil" means an oil comprising at least one silicon atom, and especially at least one Si-O group.

The term "hydrocarbon-based oil" is understood to mean an oil comprising mainly hydrogen and carbon atoms.

The term "fluorinated oil" is understood to mean an oil comprising at least one fluorine atom.

A hydrocarbon-based oil suitable for the invention can in addition optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent or agents, at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is understood to mean an oil which remains on the skin or the keratinous fibre, at room temperature and atmospheric pressure, for at least several hours and which has in particular a vapour pressure of less than $10^{-3}$ mmHg (0.13 Pa).

Hydrocarbon-based oils

Mention may in particular be made, as non-volatile hydrocarbon-based oils which can be used according to the invention, of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin seed oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810®, 812® and 818® by Dynamit Nobel,

(ii) synthetic ethers having from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR’ in which R represents a linear or branched fatty acid residue containing from 1 to 40 carbon atoms and R’ represents a hydrocarbon-based chain that is especially branched, containing from 1 to 40 carbon atoms on condition that
R + R’ is ≥10, for instance purcellin oil (cetearyl octanoate), isopropyl myristate, isopropyl palmitate, $C_{12}$-$C_{15}$ alkyl benzoate, such as the product sold under the trade name Finsolv TN® or Witconol TN® by Witco or Tegosoft TN® by Evonik Goldschmidt, 2-ethylphenyl benzoate, such as the commercial product sold under the name X-Tend 226® by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates, such as dicaprylyl carbonate, such as the product sold under the name Cetiol CC® by Cognis;

(viii) fatty amides, such as isopropyl N-lauroyl sarcosinate, such as the product sold under the trade name Eldew SL205® from Ajinomoto;
and mixtures thereof.

Preference will more particularly be given, among the non-volatile hydrocarbon oils which can be used according to the invention, to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

Mention may in particular be made, as volatile hydrocarbon oils which can be used according to the invention, of hydrocarbon oils having from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in the patent applications of Cognis WO 2007/068371 or WO 2008/155059 (mixtures of distinct alkanes differing by at least one carbon). These alkanes are obtained from fatty alcohols, themselves obtained from coconut or palm oil. Mention may be made of the mixtures of n-undecane ($C_{11}$) and n-tridecane ($C_{13}$) obtained in Examples 1 and 2 of patent application WO2008/155059 from Cognis. Mention may also be made of n-dodecane ($C_{12}$) and n-tetradecane ($C_{14}$), sold by Sasol respectively under the references Parafol 12-970 and Parafol 14-97®, and also their mixtures.

Use may also be made of other volatile hydrocarbon oils, such as petroleum distillates, in particular those sold under the name Shell Solt® by Shell. According to one embodiment, the volatile solvent is chosen from volatile hydrocarbon-based oils having from 8 to 16 carbon atoms, and mixtures thereof.

Silicone oils

The non-volatile silicone oils can be chosen in particular from non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicones, especially those with a
viscosity ≤ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

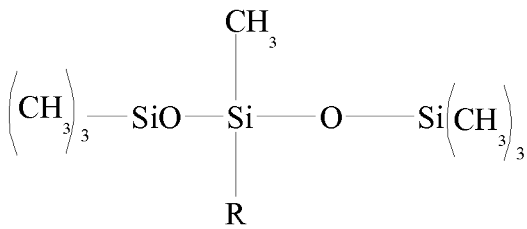

where R represents an alkyl group comprising from 2 to 4 carbon atoms, one or more hydrogen atoms of which can be replaced by a fluorine or chlorine atom.

Mention may be made, among the oils of general formula (I), of:

3-butyl-1,1,1,3,5,5,5-heptamethyltrisiloxane,
3-propyl-1,1,1,3,5,5,5-heptamethyltrisiloxane, and

3-ethyl-1,1,1,3,5,5,5-heptamethyltrisiloxane,
corresponding to the oils of formula (I) for which R is, respectively, a butyl group, a propyl group or an ethyl group.

Fluorinated oils

Use may also be made of volatile fluorinated oils, such as nonafluoromethoxybutane, decafluoropentane, tetradecafluorohexane, dodecafluoropentane, and mixtures thereof.
An oily phase according to the invention can additionally comprise other fatty substances, mixed with or dissolved in the oil.
Another fatty substance which can be present in the oily phase can be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferably, the overall oily phase, including all the lipophilic substances of the composition capable of being dissolved in this same phase, represents from 5% to 95% by weight and preferably from 10% to 80% by weight, relative to the total weight of the composition.

**AQUEOUS PHASE**

The compositions according to the invention can in addition comprise at least one aqueous phase.
The aqueous phase comprises water and optionally other water-soluble or water-miscible organic solvents.
An aqueous phase suitable for the invention can comprise, for example, a water chosen from a natural spring water, such as water from La Roche-Posay, water from Vittel or waters from Vichy, or a floral water.
The water-soluble or water-miscible solvents that are suitable for use in the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.
According to a preferred embodiment, use may more particularly be made of ethanol, propylene glycol, glycerol, and mixtures thereof.
According to a specific form of the invention, the overall aqueous phase, including all the hydrophilic substances of the composition capable of being dissolved in this same phase, represents from 5% to 95% by weight and preferably from 10% to 80% by weight, relative to the total weight of the composition.

**ADDITIVES**

**a) Additional UV-screening agents**

The compositions according to the invention may also contain one or more additional UV-screening agents chosen from hydrophilic or lipophilic organic UV-screening agents. It will preferentially consist of at least one hydrophilic, lipophilic or insoluble organic UV-screening agent.
The term "hydrophilic UV-screening agent" means any cosmetic or dermatological organic or mineral compound for screening out UV radiation, which can be fully dissolved in molecular state in a liquid aqueous phase or which can be dissolved in colloidal form (for example in micellar form) in a liquid aqueous phase.
The term "lipophilic screening agent" means any cosmetic or dermatological organic or mineral compound for screening out UV radiation, which can be fully dissolved in molecular state in a liquid fatty phase or which can be dissolved in colloidal form (for example in micellar form) in a liquid fatty phase.
The additional organic UV-screening agents are chosen in particular from cinnamic compounds; anthranilate compounds; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole derivatives; imidazoline compounds; bis-benzazolyl compounds, such as described in patents EP 669 323 and US 2 463 264; p-aminobenzoic acid (PABA) compounds; benzoxazole compounds, such as described in patent applications EP 0 832 642, EP 1 027 883, EP 1 300 137 and DE 101 62 844; screening polymers and screening silicones, such as those described in particular in patent application WO 93/04665; dimers derived from α-alkylstyrene, such as those described in patent application DE 198 55 649; 4,4-diarylbutadiene compounds, such as described in patent applications EP 0 967 200, DE 197 46 654, DE 197 55 649, EP-A-1 008 586, EP 1 133 980 and EP 133 981, and mixtures thereof.

Mention may be made, as examples of organic photoprotective agents, of those denoted hereinbelow under their INCI names:

Cinnamic compounds:

Ethylhexyl Methoxycinnamate, sold in particular under the trade name Parsol MCX® by DSM Nutritional Products,
Isopropyl Methoxycinnamate,
Isoamyl p-methoxycinnamate sold under the trade name Neo Heliopan E 1000® by Symrise,
DEA Methoxycinnamate,
Diisopropyl Methyl Cinnamate,
Glyceryl Ethylhexanoate Dimethoxycinnamate.

para-Aminobenzoic compounds:

PABA,
Ethyl PABA,
Ethyl Dihydroxypropyl PABA,
Ethylhexyl Dimethyl PABA, sold in particular under the name Escalol 507® by ISP, Glyceryl PABA,
PEG-25 PABA, sold under the name Uvinul P 25® by BASF.

Salicylic compounds:

Homosalate, sold under the name Eusolex HMS® by Rona/EM Industries,
Ethylhexyl Salicylate, sold under the name Neo Heliopan OS® by Symrise,
Dipropylene Glycol Salicylate, sold under the name Dipsal® by Scher,
TEA Salicylate, sold under the name Neo Heliopan TS® by Symrise.

β,β-Diphenylacrylate compounds:

Octocrylene, sold in particular under the trade name Uvinul N 539® by BASF, Etocrylene, sold in particular under the trade name Uvinul N 35® by BASF.

Benzophenone compounds:

Benzophenone-1, sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6, sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF, Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF.

Benzvlidenecamphor compounds:

3-Benzylidene Camphor, manufactured under the name Mexoryl SD® by Chimex, 4-Methylbenzylidene Camphor, sold under the name Eusolex 6300® by Merck,
Benzylidene Camphor Sulfonic Acid, manufactured under the name Mexoryl SL® by Chimex,
Camphor Benzalkonium Methosulfate, manufactured under the name Mexoryl SO® by Chimex,
Terephthalylidene Dicamphor Sulfonic Acid, manufactured under the name Mexoryl SX® by Chimex,
Polyacrylamidomethyl Benzylidene Camphor, manufactured under the name Mexoryl SW® by Chimex.

Phenylbenzimidazole compounds:

Phenylbenzimidazole Sulfonic Acid, sold in particular under the trade name Eusolex 232® by Merck.

Bis-benzazolyl compounds:

Disodium Phenyl Dibenzimidazole Tetrasulfonate, sold under the trade name Neo Heliopan AP® by Haarmann and Reimer.

Phenylbenzotriazole compounds:

Drometrizole Trisiloxane, sold under the name Silatrizole® by Rhodia Chimie.

Triazine compounds:

Bis-Ethylhexyloxyphenol Methoxyphenyl Triazine, sold under the trade name Tinosorb S® by BASF,
Ethylhexyl Triazone, sold in particular under the trade name Uvinul T150® by BASF,
Diethylhexyl Butamido Triazone, sold under the trade name Uvasorb HEB® by Sigma 3V,
2,4,6-tris(dineopentyl 4'-aminobenzalmalonate)-s-triazine,
2,4,6-tris(diisobutyl 4'-aminobenzalmalonate)-s-triazine,
2,4-bis(n-butyl 4'-aminobenzoate)-6-(aminopropyltrisiloxane)-s-triazine,
2,4-bis(dineopentyl 4'-aminobenzalmalonate)-6-(n-butyl 4'-aminobenzoate)-s-triazine,
silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl Anthranilate, sold under the trade name Neo Heliopan MA® by Symrise.

Imidazoline compounds:

Ethylhexyl dimethoxybenzylidene dioxoimidazoline propionate.

Benzalmalonate compounds:

Polyorganosiloxane comprising benzalmalonate functional groups, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[4-[5-(1,1-dimethylpropyl)benzoxazol-2-yl]phenylimino]-6-[(2-ethylhexyl)imino]-1,3,5-triazine, sold under the name of Uvasorb K2A® by Sigma 3V.

The preferred organic screening agents are chosen from:

Ethylhexyl methoxycinnamate,
Ethylhexyl salicylate,

Homosalate,
Octocrylene,
Phenylbenzimidazole Sulfonic Acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
Bis(ethylhexyloxyphenol)methoxyphenyltriazine,
Ethylhexyl Triazone,
Diethylhexyl Butamido Triazone,
2,4,6-tris(Dineopentyl 4'-aminobenzalmalonate)-s-triazine,
2,4,6-Tris(diisobutyl 4'-aminobenzalmalonate)-s-triazine,
2,4-Bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole trisiloxane,
Polysilicone-15,
1,1-Dicarboxy(2,2'-dimethylpropyl)-4,4-diphenylbutadiene,
2,4-Bis[4-[5-(1,1-dimethylpropyl)benzoxazol-2-yl]phenylimino]-6-[(2-ethylhexyl)imino]-1,3,5-triazine,
and mixtures thereof.

The particularly preferred organic screening agents are chosen from:

Ethylhexyl salicylate,
Homosalate,
Octocrylene,
n-Hexyl 2-(4-diethylamino-2-hydroxybenzoyl)benzoate,
Terephthalylidenedicamphorsulfonic acid,
Bis(ethylhexyloxyphenol)methoxyphenyltriazine,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole Trisiloxane,
and mixtures thereof.

The additional UV-screening agents according to the invention are preferably present in the compositions according to the invention in a content ranging from 0.1% to 45% by weight and in particular from 5% to 30% by weight relative to the total weight of the composition.

**b) Other additives:**

The compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, basifying or acidifying agents or any other ingredient commonly used in the cosmetic and/or dermatological field.

Mention may be made, among organic solvents, of alcohols other than $C_1$-$C_4$ monoalcohols as defined above and in particular short-chain $C_2$-$C_8$ polyols, such as glycerol or diols, such as caprylyl glycol, 1,2-pentanediol, propanediol, butanediol, glycols and glycol ethers, such as ethylene glycol, propylene glycol, butylene glycol, dipropylene glycol or diethylene glycol.

Mention may be made, as thickeners, of carboxyvinyl polymers, such as the Carbopols® (Carbomers) and the Pemulens, such as Pemulen TR1® and Pemulen TR2® (acrylate/$C_{10}$-$C_{30}$ alkyl acrylate copolymer); polyacrylamides, such as, for example, the crosslinked copolymers sold under the names Sepigel 305® (CTFA name: polyacryla-

mide/$C_{13-14}$ isoparaffin/Laureth 7) or Simulgel 600 (CTFA name: acrylamide/sodium acryloyldimethyl taurate copolymer/isohexadecane/polysorbate 80) by SEPPIC; 2-acrylamido-2-methylpropanesulfonic acid polymers and copolymers, optionally crosslinked and/or neutralized, such as the poly(2-acrylamido-2-methylpropanesulfonic acid) sold by Hoechst under the trade name Hostacerin AMPS® (CTFA name: ammonium polyacryloyldimethyl taurate) or Simulgel 800®, sold by SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, such as Simulgel NS® and Sepinov EMT 10®, sold by SEPPIC; cellulose derivatives, such as hydroxyethylcellulose; polysaccharides and in particular gums, such as xanthan gum; water-soluble or water-dispersible silicone derivatives, such as acrylic silicones, polyether silicones and cationic silicones, and mixtures thereof.

Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

Mention may be made, among the basifying agents, by way of example, of ammonia, alkali metal carbonates, alkanolamines, such as mono-, di- and triethanolamines and derivatives thereof, sodium hydroxide or potassium hydroxide.

Preferably, the cosmetic composition comprises one or more basifying agents selected from alkanolamines, in particular triethanolamine, and sodium hydroxide.

In the case of a direct emulsion, the pH of the composition in accordance with the invention is generally between 3 and 12 approximately, preferably between 5 and 11 approximately and more particularly still from 6 to 8.5.

Mention may be made, among the active agents for the care of keratinous substances, such as the skin, lips, scalp, hair, eyelashes or nails, of, for example:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or for preventing their decomposition;
- agents for stimulating fibroblast proliferation;
- agents for stimulating keratinocytes proliferation;
- muscle relaxants;
- refreshing agents;
- tensioning agents;
- mattifying agents;
- depigmenting agents;
- propigmenting agents;
- keratolytic agents;
- desquamating agents;
- moisturizers;
- anti-inflammatory agents;
- antimicrobial agents;
- slimming agents;
- agents acting on the energy metabolism of cells;
- insect repellents;
- substance P or CGRP antagonists;
- hair-loss counteractants;
- antiwrinkle agents;
- anti-ageing agents.

A person skilled in the art will choose said active agent(s) according to the desired effect on the skin, hair, eyelashes, eyebrows or nails.

Needless to say, a person skilled in the art will take care to choose the abovementioned optional additional

compound or compounds and/or the amounts thereof so that the advantageous properties intrinsically attached to the compositions in accordance with the invention are not, or not substantially, detrimentally affected by the envisaged addition(s).

## FORMULATION FORMS

The compositions according to the invention can be prepared according to the techniques well known to those skilled in the art. They may in particular be in the form of a simple or complex emulsion (O/W, W/O, O/W/O or W/O/W), such as a cream, a milk or a cream gel.

They can also be provided in the anhydrous form, such as, for example, in the form of an oil. The term "anhydrous composition" is understood to mean a composition comprising less than 1% by weight of water, indeed even less than 0.5% of water, and in particular devoid of water, the water not being added during the preparation of the composition but corresponding to the residual water contributed by the mixed ingredients. They can optionally be packaged as an aerosol and be provided in the form of a foam or of a spray.

In the case of compositions in the form of oil-in-water or water-in-oil emulsions, the emulsification processes which can be used are of the paddle or propeller, rotor-stator and HPH type.

In order to obtain stable emulsions with a low content of polymer (oil/polymer ratio > 25), it is possible to prepare the dispersion in concentrated phase and then to dilute the dispersion with the remainder of the aqueous phase.

It is also possible, via HPH (between 50 and 800 bar), to obtain stable dispersions with droplet sizes that may be as low as 100 nm.

The emulsions generally comprise at least one emulsifier chosen from amphoteric, anionic, cationic and nonionic emulsifiers, used alone or as a mixture. The emulsifiers are appropriately chosen according to the emulsion to be obtained (W/O or O/W).

The compositions according to the invention are preferably provided in the form of an oil-in-water or water-in-oil emulsion. The emulsifying surfactants are appropriately chosen according to the emulsion to be obtained.

Non-limiting examples of W/O emulsifying surfactants suitable for water-in-oil emulsions are given in particular in the publication entitled McCutcheon's Emulsifiers & Detergents, 1998, International Edition, MC Publishing Company, in the chapter entitled HLB Index.

Mention may be made, as examples of W/O emulsifying surfactants, of alkyl esters or ethers of sorbitan, of glycerol, of polyol or of sugars; or silicone surfactants, such as dimethicone copolyols, for example the mixture of cyclomethicone and dimethicone copolyol sold under the name DC 5225 C® by Dow Corning, and alkyl dimethicone copolyols, such as lauryl methicone copolyol, sold under the name Dow Corning 5200 Formulation Aid by Dow Corning, or cetyl dimethicone copolyol, such as the product sold under the name Abil EM 90R® by Goldschmidt and the mixture of cetyl dimethicone copolyol, polyglycerol (4 mol) isostearate and hexyl laurate sold under the name Abil WE O9® by Goldschmidt. It is also possible to add thereto one or more coemulsifiers which, advantageously, can be chosen from the group consisting of polyol alkyl esters.

Mention may also be made of non-silicone emulsifying surfactants, in particular alkyl esters or ethers of sorbitan, of glycerol, of polyol or of sugars.

Mention may in particular be made, as polyol alkyl esters, of polyethylene glycol esters, such as PEG-30 Dipolyhydroxystearate, such as the product sold under the name Arlacel P135® by ICI.

Mention may be made, as glycerol and/or sorbitan esters, for example, of polyglycerol isostearate, such as the product sold under the name Isolan GI 34® by Goldschmidt; sorbitan isostearate, such as the product sold under the name Arlacel 987® by ICI; sorbitan glyceryl isostearate, such as the product sold under the name Arlacel 986® by ICI, and mixtures thereof.

Mention may be made, for the O/W emulsions, for example, as nonionic emulsifying surfactants, of polyoxyalkylenated (more particularly polyoxyethylenated and/or polyoxypropylenated) esters of fatty acids and of glycerol; oxyalkylenated esters of fatty acids and of sorbitan; polyoxyalkylenated (in particular polyoxyethylenated and/or polyoxypropylenated) esters of fatty acids, optionally in combination with an ester of fatty acid and of glycerol, such as the PEG-100 Stearate/Glyceryl Stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by Henkel under the respective names Plantaren 2000® and Plantaren 1200®, cetearyl glucoside, optionally as a mixture with cetearyl alcohol, sold, for example, under the name Montanov 68® by SEPPIC, under the name Tegocare CG90® by Goldschmidt and under the name Emulgade KE3302® by Henkel, and arachidyl glucoside, for example in the form of the mixture of arachidyl and behenyl alcohols and of arachidyl glucoside sold under the name Montanov 202® by SEPPIC. According to a particular embodiment of the invention, the mixture of the alkyl polyglucoside as defined above with the corresponding fatty alcohol may be in the form of a self-emulsifying composition, for example as described in document WO-A-92/06778.

According to a particularly preferred form, the compositions are provided in the oil-in-water form.

When it is an emulsion, the aqueous phase of the latter can comprise a nonionic vesicular dispersion prepared according to known processes (Bangham, Standish and Watkins, J. Mol. Biol. 13, 238 (1965), FR 2 315 991 and FR 2 416 008).

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of products for the cosmetic treatment of the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a non-therapeutic cosmetic process for caring for and/or making up a keratin material, which consists in applying, to the surface of the said keratin material, at least one composition according to the invention as defined above.

The cosmetic compositions according to the invention may be used, for example, as care products and/or antisun products for the face and/or body with a liquid to semi-liquid consistency, such as milks, more or less smooth creams, cream gels or pastes. They may optionally be packaged as an aerosol and be provided in the form of a foam or of a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged as an aerosol in accordance with the invention generally comprise conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

**ASSEMBLY**

According to another aspect, the invention also relates to a cosmetic assembly comprising:

i) a container delimiting one or more compartment(s), the said container being closed by a closing member and optionally being unsealed; and

ii) a make-up and/or care composition in accordance with the invention placed inside the said compartment(s).

The container can, for example, be in the form of a pot or a case.

The closing member can be in the form of a lid comprising a cap mounted so as to be able to move by translation or by pivoting relative to the container housing the said make-up and/or care composition or compositions.

The examples which follow serve to illustrate the invention without, however, exhibiting a limiting nature. In these examples, the amounts of the composition ingredients are given as weight percentages relative to the total weight of the composition.

**Example A1: Preparation of compound (1)**

(1)

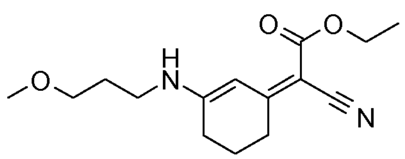

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent.

The following base/solvent combinations are used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC.
162.30 g of compound (14) were obtained in the form of a brown oil.
After crystallization, the product is obtained in the form of yellowish crystals. Melting point: 92.7°C.

**Example A2: Preparation of compound (2)**

(2)

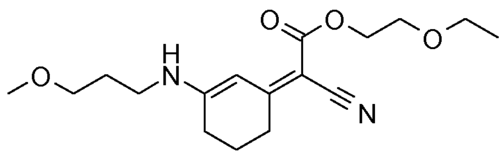

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of an organic base and a solvent.
The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | No solvent |

**Example A3 (outside the invention): Preparation of the compound (2Z)-2-cyano-N-(3-methoxypropyl)-2-{3-(3-methoxypropyl)amino]cyclohex-2-en-1-ylidene}ethanamide described in the unpublished patent application PCT/EP 2012/064 195**

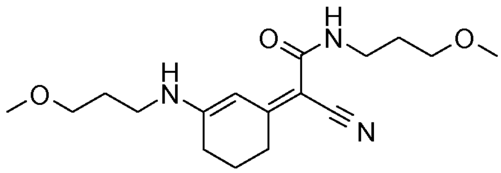

101.00 g of 3-[(3-methoxypropyl)amino]-2-cyclohexen-1-one were alkylated with dimethyl sulfate or alternatively with diethyl sulfate and treated with 86.00 g of 2-cyano-N-(3-methoxypropyl)acetamide in approximately equimolar proportions in the presence of a base and optionally of a solvent.
The following base/solvent combinations were used:

| Example | Base | Solvent |
|---|---|---|
| Example A3.1 | DBU (1,8-diazabicyclo[5.4.0]undec-7-ene) | dimethylacetamide |
| Example A3.2 | triethylamine | isopropanol |
| Example A3.3 | 3-methoxypropylamine | isopropanol |
| Example A3.4 | 3-methoxypropylamine | tert-amyl alcohol |
| Example A3.5 | 3-methoxypropylamine | toluene |
| Example A3.6 | 3-methoxypropylamine | dimethylformamide |
| Example A3.7 | 3-methoxypropylamine | no solvent |

The crude product (2Z)-2-cyano-N-(3-methoxypropyl)-2-{3-[(3-methoxypropyl)amino]cyclohex-2-en-1-ylidene} ethanamide was obtained in the form of a dark brown oil.
After chromatography on a column of silica gel (eluent: 99/1 toluene/methanol), 81.8 g of product were obtained in the form of yellowish crystals.
Melting point: 84.7-85.3°C.

## Formulation examples 1 to 9

The compound 2-ethoxyethyl (2Z)-cyano{3-[(3-methoxypropyl)amino]cyclohex-2-en-1-ylidene}ethanoate (2) of the invention was compared with:

the compound (2Z)-2-cyano-N-(3-methoxy-propyl)-2-{3-[(3-methoxypropyl)amino]cyclohex-2-en-1-ylidene}ethanamide according to Example A3 (outside the invention)
the compound octyl-5-N,N-diethylamino-2-phenysulfonyl-2,4-pentadienoate (outside the invention)
and the merocyanine compound MC11 disclosed in the application WO2008/090066 (outside of invention)

Formulations 1 to 4 below were prepared; they were constructed such that the sum of the contents of oil and of liposoluble UV-screening agents remains constant. The content of the screening agents was adjusted so as to ensure the same level of UVB screening and also the same in vitro SPF, and also the same absorbance profile between 290 and 340 nm. For each of the formulations, the SPF, the UVAPPD index and the absorbance after 24 hours at room temperature were measured. The amounts are expressed as weight percentages relative to the total weight of the composition.

| Phase | Ingredients | Formulation 1 (outside the invention) | Formulation 2 (outside the invention) |
|---|---|---|---|
| A | Water | qsp 100 | qsp 100 |
| | Glycerin | 6 | 6 |
| | Disodium EDTA | 0.1 | 0.1 |
| | Triethanolamine | 0.45 | 0.45 |
| | Methylenebis(benzotriazolyl)-tetramethylbutylphenol (Tinosorb M®) | 5% active material | 5% active material |
| | Potassium cetyl phosphate (Amphisol K®) | 1 | 1 |
| B | 2-Ethylphenyl benzoate (X-Tend 226®) | 30 | 29.4 |
| | Octyl 5-N,N-diethylamino-2-phenylsulfonyl-2,4-pentadienoate | - | 0.6 |
| | Stearic Acid | 1.5 | 1.5 |
| | Glyceryl Stearate (and) PEG-100 Stearate (Arlacel 165 ®) | 1.5 | 1.5 |
| | Dimethicone | 0.5 | 0.5 |
| | Preserving agents | 1.28 | 1.28 |
| C | Isohexadecane | 2 | 2 |
| | Xanthan gum | 0.1 | 0.1 |
| | Acrylates / C10-30 Alkyl Acrylate crosspolymer (Pemulen TR1®) | 0.25 | 0.25 |
| D | Triethanolamine | 0.25 | 0.25 |
| E | Alcohol | 2 | 2 |
| **in vitro SPF ($t_{24h}$)** | | 4.1 ± 0.3 | 3.8 ± 0.2 |
| **in vitro UVAPPD ($t_{24h}$)** | | 4.5 ± 0.3 | 6.7 ± 0.5 |

| Phase | Ingredients | Formulation 3 (outside the invention) | Formulation 4 (invention) |
|---|---|---|---|
| A | Water | qsp 100 | qsp 100 |
| | Glycerin | 6 | 6 |
| | Disodium EDTA | 0.1 | 0.1 |
| | Triethanolamine | 0.45 | 0.45 |
| | Methylenebis(benzotriazolyl)-tetramethylbutylphenol (Tinosorb M® | 5% active material | 5% active material) |
| | Potassium Cetyl Phosphate(Amphisol K®) | 1 | 1 |
| B | 2-Ethylphenyl benzoate (X-Tend 226®) | 29.2 | 29 |
| | (2Z)-2-cyano-N-(3-methoxypropyl)-2-{3-[(3-methoxypropyl)amino]cyclohex-2-en-1-ylidene}ethanamide | 0.8 | - |
| | Compound (2) | - | 1 |
| | Stearic Acid | 1.5 | 1.5 |
| | Glyceryl Stearate (and) PEG-100 Stearate (Arlacel 165®) | 1.5 | 1.5 |
| | Dimethicone | 0.5 | 0.5 |
| | Preserving agents | 1.28 | 1.28 |
| C | Isohexadecane | 2 | 2 |
| | Xanthan gum | 0.1 | 0.1 |
| | Acrylates / C10-C30 Alkyl Acrylate crosspolymer (Pemulen TR1®) | 0.25 | 0.25 |
| D | Triethanolamine | 0.25 | 0.25 |
| E | Alcohol | 2 | 2 |
| **in vitro SPF ($t_{24h}$)** | | 4.1 ± 0.1 | 4.1 ± 0.3 |
| **in vitro UVAPPD ($t_{24h}$)** | | 7.2 ± 0.3 | 7.4 ± 0.8 |

Formulations 5 a 9 below were prepared. The content of the filters was constant in order to compare the performance of the compound (2) according to the invention to the one of compound (2Z)-2-cyano-N-(3-methoxy-propyl)-2-{3-[(3-methoxypropyl)amino]cyclohex-2-en-1-ylidene}-ethanamide according to example A3 (outside the invention) and to the one of the compound MC11 disclosed in the application WO2008/090066 (outside of inventioon) at the same content. For some of these formulations the SPF, the UVAPPD index and the absorbance after one week at room temperature were measured. The amounts are expressed as weight percentages relative to the total weight of the composition

| Phase | Ingredients | Formulation 5 (outside the invention) | Formulation 6 (invention) |
|---|---|---|---|
| A | Water | qsp 100 | qsp 100 |
| | Glycerin | 5 | 5 |
| | Disodium EDTA | 0.1 | 0.1 |
| | Triethanolamine | 0.45 | 0.45 |
| | Methylene Bis-Benzotriazolyl Tetramethylbutylphenol (Tinosorb M® % in active material) | 5 | 5 |
| | Potassium Cetyl Phosphate(Amphisol K®) | 1 | 1 |
| B | Isopropyl Lauroyl Sarcosinate (Eldew SL-205®) | 30 | 30 |
| | (2Z)-2-cyano-N-(3-methoxypropyl)-2-{3-[(3-methoxypropyl)amino]cyclohex-2-en-1-ylidene}ethanamide | 2 | - |
| | Compound (2) | - | 2 |
| | Stearic acid | 1.5 | 1.5 |
| | Glyceryl Stearate (and) PEG-100 Stearate (Arlacel 165®) | 2.5 | 2.5 |
| | Dimethicone | 0.5 | 0.5 |
| | Cetyl Alcohol | 0.5 | 0.5 |
| | Cetearyl Alcohol (and) Cetearyl Glucoside (Montanov 68®) | 2 | 2 |
| | Preservatives | 1 | 1 |
| C | Isohexadecane | 1 | 1 |
| | Gomme de xanthane | 0.2 | 0.2 |
| | Acrylates / C10-C30 Alkyl Acrylate crosspolymer (Pemulen TR1®) | 0.2 | 0.2 |
| D | Triethanolamine | 0,2 | 0,2 |
| **SPF in vitro ($t_{1w}$)** | | 4.3 ± 0.6 | 3.6 ± 1.1 |
| **UVA PPD in vitro ($t_{1w}$)** | | 16.5 ± 2.2 | 11.6 ± 4.3 |

| Phase | Ingredients | Formulation 6 (invention) | Formulation 7 (outside the invention) | Formulation 8 ( outside the invention) | Formulation 9 (ouside the invention) |
|---|---|---|---|---|---|
| A | Water | qsp 100 | qsp 100 | qsp 100 | qsp 100 |
| | Glycerin | 5 | 5 | 5 | 5 |
| | Disodium EDTA | 0.1 | 0.1 | 0.1 | 0.1 |
| | Triethanolamine | 0.45 | 0.45 | 0.45 | 0.45 |
| | Methylene Bis-Benzotriazolyl Tetramethylbutylphenol (Tinosorb M® in % active material) | 5 | 5 | - | - |
| | Potassium Cetyl Phosphate(Amphisol K®) | 1 | 1 | 1 | 1 |
| B | Isopropyl Lauroyl Sarcosinate (Eldew SL-205®) | 30 | 30 | 30 | 30 |
| | Compound (2) | 2 | - | 2 | - |
| | MC11 of WO2008/090066 | - | 2 | - | 2 |
| | Stearic Acid | 1.5 | 1.5 | 1.5 | 1.5 |
| | Glyceryl Stearate (and) PEG-100 Stearate (Arlacel 165®) | 2.5 | 2.5 | 2.5 | 2.5 |
| | Dimethicone | 0.5 | 0.5 | 0.5 | 0.5 |
| | Cetyl Alcohol | 0.5 | 0.5 | 0.5 | 0.5 |
| | Cetearyl Alcohol (and) Cetearyl Glucoside (Montanov 68®) | 2 | 2 | 2 | 2 |
| | Preservatives | 1 | 1 | 1 | 1 |
| C | Isohexadecane | 1 | 1 | 1 | 1 |
| | Xanthan Gum | 0.2 | 0.2 | 0.2 | 0.2 |
| | Acrylates / C10-C30 Alkyl Acrylate crosspolymer (Pemulen TR1®) | 0.2 | 0.2 | 0.2 | 0.2 |
| D | Triethanolamine | 0.2 | 0.2 | 0.2 | 0.2 |

**Emulsion preparation method** :

The aqueous phase A and oily phase B were prepared by mixing the starting materials with mechanical stirring at 80°C. Once the aqueous solution A and oily solution B were macroscopically homogeneous, the emulsion was prepared by introducing phase B into phase A with stirring using a rotor-stator homogenizer with a stirring speed of 4500 rpm for 20 minutes. The phases C and then D were then successively added, with continued stirring. The emulsion was finally cooled to room temperature. The final emulsion was characterized by drops of between 1 μm and 20 μm in size.

**In vitro protocol for evaluating the screening efficacy**

The sun protection factor (SPF) was determined according to the in vitro method described by B.L. Diffey in J. Soc. Cosmet. Chem. 40, 127-133 (1989). The measurements were carried out using a UV-1000S spectrophotometer from the company Labsphere. The "static in vitro protection factor (SPF)" value is extracted. Each composition is applied to a rough plate of PMMA in the form of a uniform and even deposit in a proportion of 1 mg/cm2.
The in vitro UVAPPD index measurements were taken under the same conditions using a UV-1000S spectrophotometer from the company Labsphere. The "UVAPPD index (persistent pigment darkening action spectrum)" value is extracted. Each composition is applied to a rough plate of PMMA, in the form of a uniform and even deposit at a rate of 1 mg/cm2.

**Protocol for evaluating the absorbance spectra of the formulations**

The absorbance spectra of the formulations were extracted from the mAF data as a function of the wavelength generated during the in vitro SPF measurement and the in vitro PPD measurement. The mAF values were then converted into absorbance values according to: Abs= log(mAF). The profiles may be normalized to 320 nm.

**Absorbance of the formulations measured 24 hours after formulation**

| Absorbance | Formulation 1 (outside the invention) | Formulation 2 (outside the invention) | Formulation 3 (outside the invention) | Formulation 4 (invention) |
|---|---|---|---|---|
| Absorbance at 290 nm after 24 hours | 0.61 ± 0.02 | 0.57 ± 0.03 | 0.59 ± 0.01 | 0.58 ± 0.03 |
| Absorbance at 320 nm after 24 hours | 0.60 ± 0.03 | 0.56 ± 0.02 | 0.60 ± 0.01 | 0.59 ± 0.04 |
| Absorbance at 400 nm after 24 hours | 0.24 ± 0.01 | 0.28 ± 0.01 | 0.54 ± 0.01 | **0.65 ± 0.04** |

**Conclusions**

The in vitro UVAPPD index values for formulation 1 show that for the same SPF and a similar absorbance profile in the UVB range, from 290 to 320 nm (Formulation 2), Tinosorb M® alone is less protecting than the Tinosorb M®/merocyanines combinations (Formulations 2, 3 and 4).
The Tinosorb M®/Compound 2 combination (Formulation 4) of the invention differs from the Tinosorb M®/Octyl 5-N,N-diethylamino-2-phenysulfonyl-2,4-pentadienoate (Formulation 2) and Tinosorb M ®/(2Z)-2-cyano-N-(3-methoxypropyl)-2-{3-[(3-methoxypropyl)amino]cyclohex-2-en-1-ylidene}ethanamide (Formulation 3) couples by a broader absorbance profile in the UVA range and a significantly higher absorbance at 400 nm for comparable in vitro SPF and UVAPPD values.

**Absorbance of the formulations 5 and 6 measured after storage of 1 week at room temperature and 45 days at 45°C**

| Absorbance | | Formulation 5 (outside the invention) | Formulation 6 (invention) |
|---|---|---|---|
| Absorbance after 1 week at room temperature | at 290 nm<br>at 320 nm<br>at 400 nm | 0.51 ± 0.05<br>0.65 ± 0.05<br>0.84 ± 0.02 | 0.46 ± 0.12<br>0.55 ± 0.13<br>0.90 ± 0.13 |
| Absorbance after 45 days at 45°C | at 400 nm | 0.80 ± 0.09 | **0.98 ± 0.01** |

The Tinosorb M®/Compound 2 combination (Formulation 6) of the invention differs from the Tinosorb M ®/ (2Z)-2-cyano-N-(3-methoxypropyl)-2-{3-[(3-methoxypropyl)amino]cyclohex-2-en-1-ylidene}-ethanamide combination (Formulation 5) by a significantly higher absorbance at 400 nm after 45 days of storage at 45°C for comparable in vitro SPF and UVAPPD values at 1 week.

**Protocol for evaluating the color of the formulations**

The color of the formulations was evaluated after preparation of thin films on contrast map. The formulations were deposited within a circle of 2.2 cm of diameter and planed to obtain thicknesses of reproducible deposit. The colorimetric measures were then made by means of a spectro-colorimeter Minolta CM2600D in two points of the film. This operation is twice reproduced, which leads to 4 experimental values by formulation.

The results are expressed in the system (L *, has *, b *) in which L* represents the luminance, a* represents the red-green axis (-a* = green, +a* = red) and b* represents the yellow-blue axis (-b* blue, +b* yellow). So, a* and b* express the shade of the compound.

The difference of color ΔE* was calculated from the variations L*, a* et b* between the compound (2) and the compound MC11 with the following equation :

$$(\Delta E^*)^2 = (\Delta L^*)^2 + (\Delta a^*)^2 + (\Delta b^*)^2$$

$$\Delta L^* = L^*_{\text{formulation with compound MC11}} - L^*_{\text{formulation with compound (2)}}$$

$$\Delta a^* = a^*_{\text{formulation with compound MC11}} - a^*_{\text{formulation with compound (2)}}$$

$$\Delta b^* = b^*_{\text{formulation with compound MC11}} - b^*_{\text{formulation with compound (2)}}$$

We consider that the difference of color between the two compounds is significant if ΔE* > 2.

**Colorimetric measures on the formulations 6 to 9**

| | Formulation 6 (invention) | Formulation 7 (outside the invention) | Formulation 8 (outside the invention) | Formulation 9 (outside the invention) |
|---|---|---|---|---|
| L* | 93.8 ± 0.7 | 93.2 ± 0.9 | 93.1 ± 0.6 | 93.0 ± 0.6 |
| a* | -4.06 ± 0.04 | -4.62 ± 0.5 | -5.08 ± 0,03 | -6.40 ± 0.05 |
| Δa * | - 0,56 | | -1,32 | |
| b* | 8.66 ± 0,05 | 11.6 ± 0.2 | 12.0 ± 0.2 | 15.8 ± 0.2 |
| **Δb *** | **2.9** | | **3.8** | |
| **ΔE *** | **3.0** | | **4.0** | |

The colorimetry results on the examples 6 to 9 show that the formulations 6 and 8 with the compound (2) are significantly less yellow than the equivalent formulations 7 and 9 with the compound MC11 of the application WO2008/ 090066.

**Formulations 10 to 13**

The compound 2-ethoxyethyl (2Z)-cyano{3-[(3-methoxypropyl)amino]cyclohex-2-en-1-ylidene}ethanoate (2) of the invention was compared with:

the compound (2Z)-2-cyano-N-(3-methoxy-propyl)-2-{3-[(3-methoxypropyl)amino]cyclohex-2-en-1-ylidene}ethanamide according to Example A3 (outside the invention)
the compound octyl-5-N,N-diethylamino-2-phenysulfonyl-2,4-pentadienoate (outside the invention)
and the merocyanine compound MC11 disclosed in the application WO2008/090066 (outside the invention)

Formulae 10 to 12 below were prepared : they were constructed such that the sum of the contents of oil and of liposoluble UV-screening agents remains constant. The content of the screening agents was adjusted so as to ensure the same level of UVB screening and also the same in vitro SPF, and also the same absorbance profile between 290 and 340 nm. For each of the formulae, the SPF, the UVAPPD index and the absorbance after 24 hours at room temperature and after 10 days at 60°C were measured. The amounts are expressed as weight percentages relative to the total weight of the composition.

| Phase | Ingredients | Formulation 10 (outside invention) | Formulation 11 (outside the invention) | Formulation 12 (invention) | Formulation 13 (outside the invention) |
|---|---|---|---|---|---|
| A | Water | qsp 100 | qsp 100 | qsp 100 | qsp 100 |
| | Glycerin | 6 | 6 | 6 | 6 |
| | Disodium EDTA | 0.1 | 0.1 | 0.1 | 0.1 |
| | Triethanolamine | 0.45 | 0.45 | 0.45 | 0.45 |
| | Potassium Cetyl Phosphate (Amphisol K®) | 1 | 1 | 1 | 1 |
| B | 2-Ethylphenyl Benzoate (X-Tend 226 ®) | 22.7 | 22.8 | 22.6 | 22.6 |
| | TITANIUM DIOXIDE (and) ALUMINUM HYDROXIDE (and) STEARIC ACID (MICRO TITANIUM DIOXIDE MT-100 TV ® | 6 | 6 | 6 | 6 |
| | - TAYCA) | | | | |
| | Octyl-5-N,N-diethylamino-2-phenysulfonyl-2,4-pentad ienoate | 1.3 | - | - | - |
| | (2Z)-2-cyano-N-(3-methoxypropyl)-2-{3-[(3-methoxypropyl)amino]c yclohex-2-en-1-ylidene} ethanamide | - | 1.2 | - | - |
| | Compound (2) | - | - | 1.4 | - |
| | MC11 of WO2008/090066 | - | - | - | 1.4 |
| | Stearic Acid | 1.5 | 1.5 | 1.5 | 1.5 |
| | Glyceryl Stearate (and) PEG-100 Stearate (Arlacel 165®) | 1.5 | 1.5 | 1.5 | 1.5 |
| | Dimethicone | 0.5 | 0.5 | 0.5 | 0.5 |
| | Preservatives | 1.28 | 1.28 | 1.28 | 1.28 |

(continued)

| Phase | Ingredients | Formulation 10 (outside invention) | Formulation 11 (outside the invention) | Formulation 12 (invention) | Formulation 13 (outside the invention) |
|---|---|---|---|---|---|
| C | Isohexadecane | 2 | 2 | 2 | 2 |
| | Xanthan gum | 0.1 | 0.1 | 0.1 | 0.1 |
| | Acrylates / C10-C30 Alkyl Acrylate crosspolymer (Pemulen TR1®) | 0.25 | 0.25 | 0.25 | 0.25 |
| D | Triethanolamine | 0.25 | 0.25 | 0.25 | 0.25 |
| E | Alcohol | 2 | 2 | 2 | 2 |
| **SPF in vitro ($t_{24h}$) UVAPPD in vitro ($t_{24h}$)** | | $5.6 \pm 0.1$<br>$7.1 \pm 0.6$ | $5.4 \pm 0.7$<br>$6.0 \pm 0.1$ | $5.7 \pm 0.4$<br>$6.7 \pm 0.6$ | |
| **SPF in vitro ($t_{10d}$ at 60°C) UVAPPD in vitro ($t_{10d}$ at 60°C)** | | - | $6.7 \pm 1.6$<br>$5.9 \pm 0.9$ | $5.9 \pm 0.8$<br>$6.3 \pm 0.5$ | |

Emulsions 10 to 13 were prepared according to the same preparation mode as for Examples 1 to 9.

The in vitro SPF and UVAPPD index values were measured under the same conditions indicated previously.

**Colorimetric measures on the formulations 12 and 13**

The colorimetric measures were made on the formulations 12 and 13 in the same conditions as previously indicated

| | Formulation 12 (invention) | Formulation 13 (outside the invention) |
|---|---|---|
| L* | $93.2 \pm 0.4$ | $93.2 \pm 0.5$ |
| ΔL* | | |
| a* | $-4.65 \pm 0.02$ | $-5{,}72 \pm 0.09$ |
| Δa* | -1.08 | |
| b* | $11.8 \pm 0.1$ | $14.5 \pm 0.3$ |
| **Δb*** | **2.75** | |
| **ΔE*** | **2.97** | |

The colorimetry results on the examples 12 and 13 show that the formulation 12 with the compound (2) is significantly less yellow than the equivalent formulation 13 with the compound MC11 of the application WO2008/090066.

**Claims**

**1.** Cosmetic or dermatological composition comprising, in a physiologically acceptable support:

a) at least one oily phase and
b) at least one merocyanine compound corresponding to formula (1) below, and also the E/E- or E/Z- geometrical isomer forms thereof:

(1) 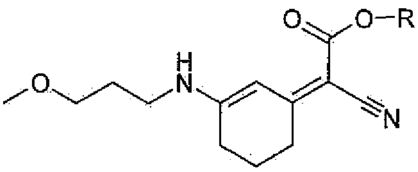

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being substituted with one or more O, and

c) at least one insoluble organic UV-screening agent and/or one insoluble inorganic UV-screening agent.

**2.** Composition according to Claim 1, in which the merocyanine compound(s) of formula (1) are chosen from those in which:

R is a $C_1$-$C_{22}$ alkyl, which may be substituted with one or more O.

**3.** Composition according to Claim 1 or 2, in which the merocyanine compound(s) of formula (1) are chosen from the following compounds, and also the E/E- or E/Z-geometrical isomer forms thereof:

| | | | |
|---|---|---|---|
| 1. | 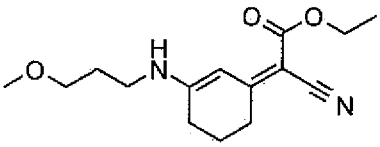 ethyl (2Z)-cyano{3-[{3-methoxypropyl)amino] cyclohex-2-en-1-ylidene}ethanoate | 4 | 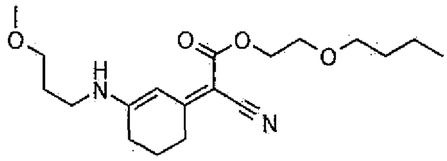 2-butoxyethyl (2Z)-cyano{3-[(3-methoxypropyl) amino]cyclohex-2-en-1-ylidene}ethanoate |
| 2. | 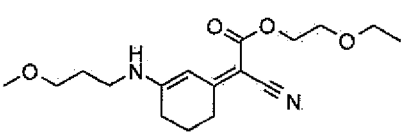 2-ethoxyethyl (2Z)-cyano{3-[(3-methoxypropyl) amino]cyclohex-2-en-1-ylidene}ethanoate | 5 | 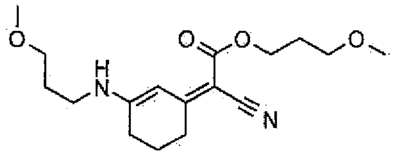 3-methoxypropyl (2Z)-cyano{3-[(3-methoxypropyl) amino]cyclohex-2-en-1-ylidene}ethanoate |
| 3 | 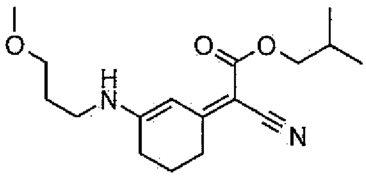 2-methylpropyl (2Z)-cyano{3-[(3-methoxypropyl) amino]cyclohex-2-en-1-ylidene}ethanoate | 6 | 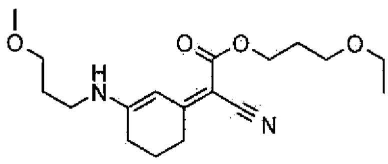 3-ethoxypropyl (2Z)-cyano{3-[(3-methoxypropyl) amino]cyclohex-2-en-1-ylidene}ethanoate |

**4.** Composition according to Claim 3, in which the merocyanine compound is 2-ethoxyethyl (2Z)-cyano{3-[(3-methoxypropyl)amino]cyclohex-2-en-1-ylidene}ethanoate (2) in its E/Z geometrical configuration having the following structure:

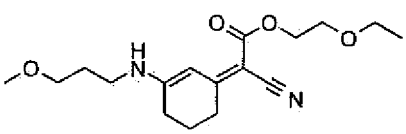

and/or in its E/E geometrical configuration having the following structure:

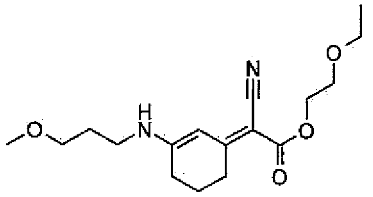

5. Composition according to any one of Claims 1 to 4, in which the merocyanine compound(s) of formula (1) are present in a concentration ranging from 0.1% to 10% by weight and preferentially from 0.2% to 5% by weight relative to the total weight of the composition.

6. Composition according to any one of Claims 1 to 5, in which the insoluble organic UV-screening agent is in the form of particles with a mean size ranging from 0.01 to 5 μm, more preferentially from 0.01 to 2 μm and more particularly from 0.020 to 2 μm.

7. Composition according to any one of Claims 1 to 6, in which the insoluble organic UV-screening agent is chosen from organic UV-screening agents of the oxalanilide type, of the triazine type, of the benzotriazole type; of the vinylamide type; of the cinnamide type; of the type comprising one or more benzazole and/or benzofuran, benzothiophenene groups or of the indole type; of the aryl vinylene ketone type; of the phenylene bis-benzoxazinone derivative type, or of the amide, sulfonamide or acrylonitrile carbamate derivative type, or mixtures thereof.

8. Composition according to any one of Claims 1 to 7, in which the insoluble organic UV-screening agent is chosen from:

(i) symmetrical triazines substituted with naphthalenyl groups or polyphenyl groups, in particular 2,4,6-tris(diphenyl)triazine and/or 2,4,6-tris(terphenyl)triazine;
(ii) the methylenebis(hydroxyphenylbenzotriazole) compounds of formula (IV) below:

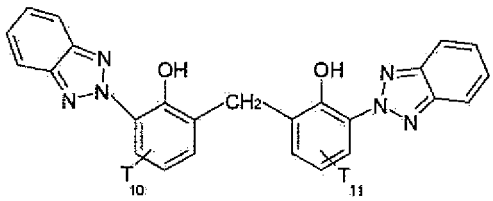

(IV)

in which the radicals $T_{10}$ and $T_{11}$, which may be identical or different, denote a $C_1$-$C_{18}$ alkyl radical possibly substituted with one or more radicals chosen from $C_1$-$C_4$ alkyl and $C_5$-$C_{12}$ cycloalkyl or an aryl residue;
(iii) and mixtures thereof.

9. Composition according to Claim 8, in which the methylenebis(hydroxyphenylbenzotriazole) compound of formula (IV) is in the form of an aqueous dispersion of particles with a mean particle size which ranges from 0.01 to 5 μm, more preferentially from 0.01 to 2 μm and more particularly from 0.020 to 2 μm in the presence of at least one surfactant of structure $C_nH_{2n+1}$ $O(C_6H_{10}O_5)_xH$ in which n is an integer from 8 to 16 and x is the average degree of polymerization of the unit $(C_6H_{10}O_5)$ and ranges from 1.4 to 1.6.

10. Composition according to Claim 8 or 9, in which the methylenebis(hydroxyphenylbenzotriazole) compound of formula (IV) is in the form of an aqueous dispersion of particles with a mean size ranging from 0.02 to 2 μm, more preferentially from 0.01 to 1.5 μm and more particularly from 0.02 to 1 μm, in the presence of at least one polyglyceryl mono($C_8$-$C_{20}$)alkyl ester with a degree of glycerol polymerization of at least 5.

11. Composition according to Claim 10, in which the polyglyceryl mono($C_8$-$C_{20}$)alkyl ester is chosen from decaglyceryl caprate, decaglyceryl laurate, decaglyceryl myristate, decaglyceryl oleate, decaglyceryl stearate, decaglyceryl isostearate, hexaglyceryl caprate, hexaglyceryl laurate, hexaglyceryl myristate, hexaglyceryl oleate, hexaglyceryl stearate, hexaglyceryl isostearate, pentaglyceryl caprate, pentaglyceryl laurate, pentaglyceryl myristate, pentaglyceryl oleate, pentaglyceryl stearate and pentaglyceryl isostearate, and more particularly from decaglyceryl caprate and

decaglyceryl laurate.

**12.** Composition according to Claim 10 or 11, in which the amount of methylenebis(hydroxyphenylbenzotriazole) compound of formula (IV) in the aqueous dispersion ranges from 10% to 50% by weight and more preferentially from 30% to 50% by weight relative to the total weight of the dispersion.

**13.** Composition according to any one of Claims 10 to 12, in which the weight ratio of methylenebis(hydroxyphenylbenzotriazole) compound/polyglyceryl mono($C_8$-$C_{20}$)alkyl ester ranges from 0.05 to 0.5 and more preferentially from 0.1 to 0.3.

**14.** Composition according to any one of Claims 9 to 13, in which the methylenebis(hydroxyphenylbenzotriazole) compound of formula (IV) in aqueous dispersion form is the compound 2,2'-methylenebis[6-(2H-benzotriazol-2-yl)-4-(1,1,3,3-tetramethylbutyl)phenol] having the following structure:

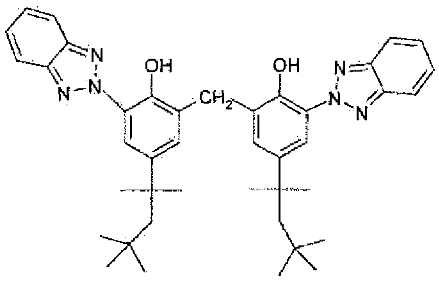

compound (a)

**15.** Composition according to any one of Claims 1 to 14, in which the insoluble organic UV-screening agent(s) of the invention are present in an active material concentration ranging from 0.1% to 15% by weight and more particularly from 0.5% to 10% by weight relative to the total weight of the composition.

**16.** Composition according to any one of Claims 1 to 5, in which the insoluble inorganic UV-screening agent is chosen from coated or uncoated metal oxide pigments, chosen especially from titanium, zinc, iron, zirconium and cerium oxides, or mixtures thereof.

**17.** Composition according to Claim 16, in which the metal oxide particles have a mean elementary particle size of less than or equal to 0.5 μm, more preferentially between 0.005 and 0.5 μm, even more preferentially between 0.01 and 0.2 μm, better still between 0.01 and 0.1 μm and more particularly preferentially between 0.015 and 0.05 μm.

**18.** Composition according to Claim 16 or 17, in which the insoluble mineral UV-screening agent consists of coated or uncoated titanium dioxide particles.

**19.** Composition according to any one of Claims 1 to 5, and 16 to 18, in which the insoluble inorganic UV-screening agent(s) are present in contents ranging from 0.1% to 30% by weight, more particularly from 0.5% to 20% by weight and in particular from 1% to 10% by weight, relative to the total weight of the composition.

**20.** **Composition as defined in any one of the preceding claims** for use in a method for caring for and/or making up a keratin material, comprising the application to the surface of the said keratin material of **the said composition.**

**21.** **Composition as defined in any one of the preceding claims** for use in a method for limiting the darkening of the skin and/or improving the colour and/or the uniformity of the complexion, comprising the application to the surface of the skin of **the said composition.**

**22.** **Composition as defined in any one of the preceding claims** for use in a method for preventing and/or treating the signs of ageing of a keratin material, comprising the application to the surface of the keratin material of **the said composition.**

## Patentansprüche

**1.** Kosmetische oder dermatologische Zusammensetzung, umfassend in einem physiologisch unbedenklichen Träger:

a) mindestens eine ölige Phase und

b) mindestens eine Merocyanin-Verbindung der nachstehenden Formel (1) sowie die geometrischen E/E- oder E/Z-Isomerformen davon:

(1) 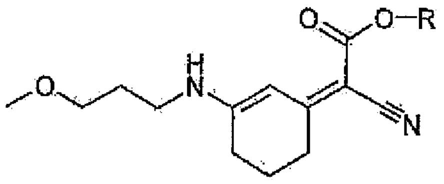

wobei:

R für eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenyl-gruppe steht, wobei die Gruppen gegebenenfalls durch ein oder mehrere 0 substituiert sein können, und

c) mindestens eine unlösliche organische UV-Filtersubstanz und/oder eine unlösliche anorganische UV-Filtersubstanz.

**2.** Zusammensetzung nach Anspruch 1, wobei die Merocyanin-Verbindung(en) der Formel (1) aus denjenigen ausgewählt sind, in denen:

R für ein $C_1$-$C_{22}$-Alkyl, das durch ein oder mehrere O substituiert sein kann, steht.

**3.** Zusammensetzung nach Anspruch 1 oder 2, wobei die Merocyanin-Verbindung(en) der Formel (1) aus den folgenden Verbindungen sowie den geometrischen E/E- oder E/Z-Isomerformen davon ausgewählt sind:

| | | | |
|---|---|---|---|
| 1 | 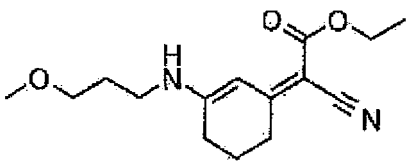<br>(2Z)-Cyano{3-[(3-methoxypropyl}amino]-cyclohex-2-en-1-yliden}ethansäure-ethylester | 4 | 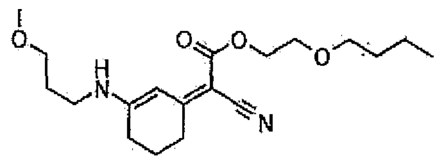<br>(2Z)-Cyano{3-[(3-methoxypropyl)amino]-cyclohex-2-en-1-yliden}ethansäure-2-butoxyethylester |
| 2 | 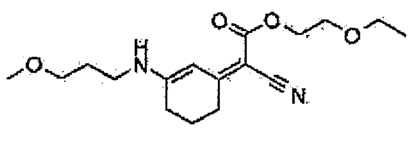<br>(2Z)-Cyano{3-[(3-methoxypropyl)amino]-cyclohex-2-en-1-yliden}ethansäure-2-ethoxyethylester | 5 | 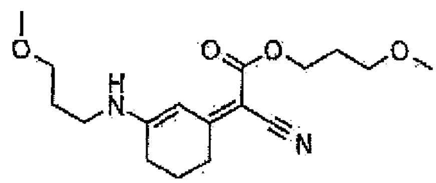<br>(2Z)-Cyano{3-[(3-methoxypropyl)amino]-cyclohex-2-en-1-yliden}ethansäure-3-methoxypropylester |
| 3 | 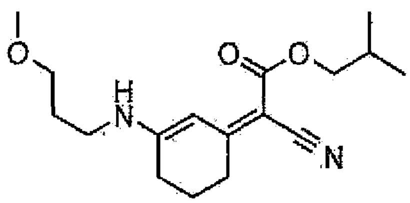<br>(2Z)-Cyano{3-[(3-methoxypropyl)amino]-cyclohex-2-en-1-yliden}ethansäure-2-methylpropylester | 6 | 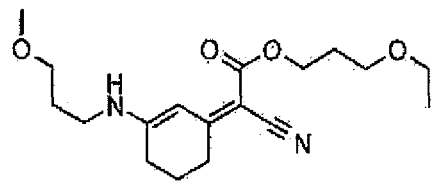<br>(2Z)-Cyano{3-[(3-methoxypropyl)amino]-cyclohex-2-en-1-yliden}ethansäure-3-ethoxypropylester |

**4.** Zusammensetzung nach Anspruch 3, wobei es sich bei der Merocyanin-Verbindung um (2Z)-Cyano{3-[(3-methoxypropyl)amino]cyclohex-2-en-1-yliden}ethan-säure-2-ethoxyethylester (2) in seiner geometrischen E/Z-Konfiguration mit der folgenden Struktur:

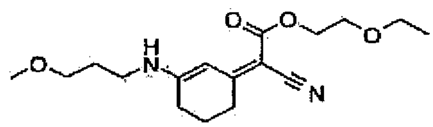

und/oder in seiner geometrischen E/E-Konfiguration mit der folgenden Struktur:

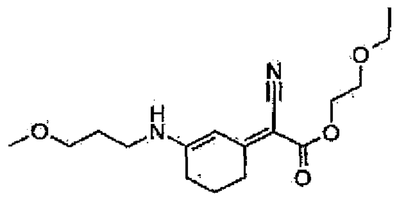

handelt.

**5.** Zusammensetzung nach einem der Ansprüche 1 bis 4, wobei die Merocyanin-Verbindung(en) der Formel (1) in einer Konzentration im Bereich von 0,1 bis 10 Gew.-% und vorzugsweise von 0,2 bis 5 Gew. -%, bezogen auf das Gesamtgewicht der Zusammensetzung, vorliegt bzw. vorliegen.

**6.** Zusammensetzung nach einem der Ansprüche 1 bis 5, wobei die unlösliche organische UV-Filtersubstanz in Form von Teilchen in einer mittleren Größe im Bereich von 0,01 bis 5 μm, weiter bevorzugt von 0, 01 bis 2 μm und spezieller von 0,020 bis 2 μm vorliegt.

**7.** Zusammensetzung nach einem der Ansprüche 1 bis 6, wobei die unlösliche organische UV-Filtersubstanz aus organischen UV-Filtersubstanzen vom Oxalanilid-Typ, vom Triazin-Typ, vom Benzotriazol-Typ, vom Vinylamid-Typ, vom Cinnamid-Typ, vom Typ, der ein oder mehrere Benzazol- und/oder Benzofuran- öder Benzothiophengruppen umfasst, oder vom Indol-Typ, vom Arylvinylenketon-Typ, vom Phenylenbisbenzoxazinonderivat-Typ oder vom Amid-, Sulfonamid- oder Acrylnitrilcarbamatderivat-Typ oder Mischungen davon ausgewählt ist.

**8.** Zusammensetzung nach einem der Ansprüche 1 bis 7, wobei die unlösliche organische UV-Filtersubstanz aus:

(i) symmetrischen Triazinen, die durch Naphthalinylgruppen oder Polyphenylgruppen substituiert sind, insbesondere 2,4,6-Tris-(diphenyl)triazin und/oder 2,4,6-Tris(terphenyl)-triazin;
(ii) den Methylenbis(hydroxyphenylbenzotriazol)-Verbindungen der nachstehenden Formel (IV):

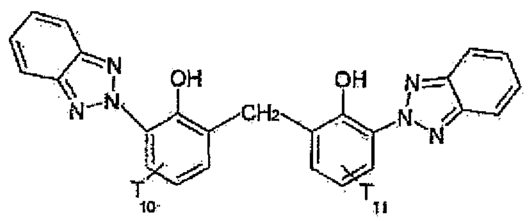

(IV)

wobei die Reste Tio und $T_{11}$, die gleich oder verschieden sein können, für einen $C_1$-$C_{18}$-Alkylrest, der gegebenenfalls durch einen oder mehrere Reste, die aus $C_1$-$C_4$-Alkyl, $C_5$-$C_{12}$-Cycloalkyl und einem Arylrest ausgewählt sind, substituiert ist, stehen;
(iii) und Mischungen davon
ausgewählt ist.

**9.** Zusammensetzung nach Anspruch 8, wobei die Methylenbis(hydroxyphenylbenzotriazol)-Verbindung der Formel (IV) in Form einer wässrigen Dispersion von Teilchen mit einer mittleren Teilchengröße im Bereich von 0,01 bis 5 μm, weiter bevorzugt von 0,01 bis 2 μm und spezieller von 0,020 bis 2 μm in Gegenwart von mindestens einem Tensid der Struktur $C_nH_{2n+1}O(C_6H_{10}O_5)_xH$, wobei n für eine ganze Zahl von 8 bis 16 steht und x für den durchschnittlichen Polymerisationsgrad der Einheit ($C_6H_{10}O_5$) steht und im Bereich von 1,4 bis 1,6 liegt, vorliegt.

**10.** Zusammensetzung nach Anspruch 8 oder 9, wobei die Methylenbis(hydroxyphenylbenzotriazol)-Verbindung der Formel (IV) in Form einer wässrigen Dispersion von Teilchen mit einer mittleren Größe im Bereich von 0,02 bis 2 µm, weiter bevorzugt von 0,01 bis 1,5 µm und weiter bevorzugt von 0,02 bis 1 µm in Gegenwart von mindestens einem Polyglycerylmono(Cs-$C_{20}$)alkylester mit einem Glycerin-Polymerisationsgrad von mindestens 5 vorliegt.

**11.** Zusammensetzung nach Anspruch 10, wobei der Polyglycerylmono($C_8$-$C_{20}$)alkylester aus Decaglycerylcaprat, Decaglyceryllaurat, Decaglycerylmyristat, Decaglyceryloleat, Decaglycerylstearat, Decaglycerylisostearat, Hexaglycerylcaprat, Hexaglyceryllaurat, Hexaglycerylmyristat, Hexaglyceryloleat, Hexaglycerylstearat, Hexaglycerylisostearat, Pentaglycerylcaprat, Pentaglyceryllaurat, Pentaglycerylmyristat, Pentaglyceryloleat, Pentaglycerylstearat und Pentaglycerylisostearat und spezieller aus Decaglycerylcaprat und Decaglyceryllaurat ausgewählt ist.

**12.** Zusammensetzung nach Anspruch 10 oder 11, wobei die Menge der Methylenbis(hydroxyphenylbenzo-triazol)-Verbindung der Formel (IV) in der wässrigen Dispersion im Bereich von 10 bis 50 Gew.-% und weiter bevorzugt von 30 bis 50 Gew.-%, bezogen auf das Gesamtgewicht der Dispersion, liegt.

**13.** Zusammensetzung nach einem der Ansprüche 10 bis 12, wobei das Gewichtsverhältnis von Methylen-bis(hydroxyphenylbenzotriazol)-Verbindung zu Polyglycerylmono ($C_8$-$C_{20}$) alkylester im Bereich von 0,05 bis 0,5 und weiter bevorzugt von 0,1 bis 0,3 liegt.

**14.** Zusammensetzung nach einem der Ansprüche 9 bis 13, wobei es sich bei der Methylenbis(hydroxyphenyl-benzotriazol)-Verbindung der Formel (IV) in Form einer wässrigen Dispersion um die Verbindung 2,2'-Methylenbis[6-(2H-benzotriazol-2-yl)-4-(1,1,3,3-tetramethylbutyl)phenol] mit der folgenden Struktur handelt:

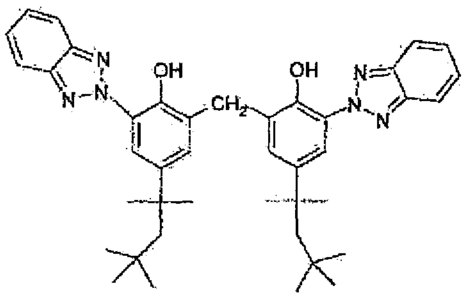

Verbindung (a)

**15.** Zusammensetzung nach einem der Ansprüche 1 bis 14, wobei die unlösliche organische UV-Filtersubstanz bzw. die unlöslichen organischen UV-Filtersubstanzen der Erfindung in einer Wirkstoffkonzentration im Bereich von 0,1 bis 15 Gew.-% und spezieller von 0,5 bis 10 Gew.-%, bezogen auf das Gesamtgewicht der Zusammensetzung, vorliegt bzw. vorliegen.

**16.** Zusammensetzung nach einem der Ansprüche 1 bis 5, wobei die unlösliche anorganische UV-Filtersubstanz aus beschichteten oder unbeschichteten Metalloxidpigmenten, die insbesondere aus Titan-, Zink-, Eisen-, Zirconium- und Ceroxiden ausgewählt sind, oder Mischungen davon ausgewählt ist.

**17.** Zusammensetzung nach Anspruch 16, wobei die Metalloxidteilchen eine mittlere Elementarteilchengröße kleiner gleich 0,5 µm, weiter bevorzugt zwischen 0,005 und 0,5 µm, noch weiter bevorzugt zwischen 0,01 und 0,2 µm, noch besser zwischen 0,01 0,1 µm und spezieller bevorzugt zwischen 0,015 und 0,05 µm aufweisen.

**18.** Zusammensetzung nach Anspruch 16 oder 17, wobei die unlösliche mineralische UV-Filtersubstanz aus beschichteten oder unbeschichteten Titandioxidteilchen besteht.

**19.** Zusammensetzung nach einem der Ansprüche 1 bis 5 und 16 bis 18, wobei die unlösliche anorganische UV-Filtersubstanz bzw. die unlöslichen organischen UV-Filtersubstanzen in Gehalten im Bereich von 0,1 bis 30 Gew.-%, speziell von 0,5 bis 20 Gew.-% und insbesondere von 1 bis 10 Gew.-%, bezogen auf das Gesamtgewicht der Zusammensetzung, vorliegt bzw. vorliegen.

**20.** Zusammensetzung gemäß einem der vorhergehenden Ansprüche zur Verwendung bei einem Verfahren zum Pflegen und/oder Schminken eines Keratinmaterials, bei dem man die Zusammensetzung auf die Oberfläche des Keratinmaterials aufbringt.

**21.** Zusammensetzung gemäß einem der vorhergehenden Ansprüche zur Verwendung bei einem Verfahren zum Einschränken des Dunkelwerdens der Haut und/oder zum Verbessern der Farbe und/oder Einheitlichkeit des Teints, bei dem man die Zusammensetzung auf die Oberfläche der Haut aufbringt.

**22.** Zusammensetzung gemäß einem der vorhergehenden Ansprüche zur Verwendung bei einem Verfahren zum Verhindern und/oder Behandeln der Anzeichen von Alterung eines Keratinmaterials, bei dem man die Zusammensetzung auf die Oberfläche des Keratinmaterials aufbringt.

## Revendications

**1.** Composition cosmétique ou dermatologique, comprenant, dans un support physiologiquement acceptable,

a) au moins une phase huileuse, et
b) au moins un composé de mérocyanine correspondant à la formule (1) ci-après, ainsi que les formes d'isomères géométriques E/E ou E/Z de celui-ci :

(1) 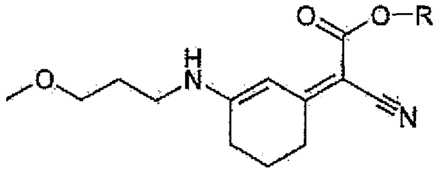

où
R est un groupement $C_1$-$C_{22}$-alkyle, un groupement $C_2$-$C_{22}$-alcényle, un groupement $C_2$-$C_{22}$-alcynyle, un groupement $C_3$-$C_{22}$-cycloalkyle ou un groupement $C_3$-$C_{22}$-cycloalcényle, lesdits groupements étant éventuellement substitués par un ou plusieurs O, et
c) au moins un agent anti-UV organique insoluble et/ou un agent anti-UV inorganique insoluble.

**2.** Composition selon la revendication 1, dans laquelle le ou les composés de mérocyanine de formule (1) sont choisis parmi ceux dans lesquels
R est $C_1$-$C_{22}$-alkyle, pouvant être substitué par un ou plusieurs O.

**3.** Composition selon la revendication 1 ou 2, dans laquelle le ou les composés de mérocyanine de formule (1) sont choisis parmi les composés suivants, ainsi que les formes d'isomères géométriques E/E ou E/Z de ceux-ci :

| | | | |
|---|---|---|---|
| 1. | 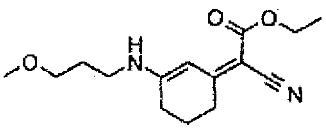<br>(2Z)-cyano-{3-[(3-méthoxy-propyl) amino] cyclohex-2-én-1-ylidène}éthanoate d'éthyle | 4. | 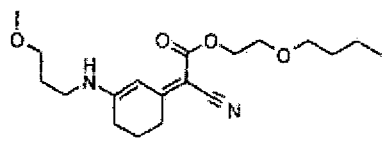<br>(2Z)-cyano-{3-[(3-méthoxy-propyl)amino] cyclohex-2-én-1-ylidène}éthanoate de 2-butoxyéthyle |
| 2. | 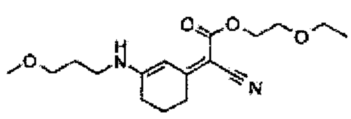<br>(2Z)-cyano-{3-[(3-méthoxy-propyl)amino]cyclohex-2-én-1-ylidène}éthanoate de 2-éthoxyéthyle | 5. | 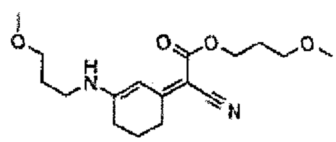<br>(2Z) -cyano-{3-[(3-méthoxy-propyl) amino] cyclohex-2-én-1-ylidène}éthanoate de 3-méthoxypropyle |

(suite)

| | | | |
|---|---|---|---|
| 3. | 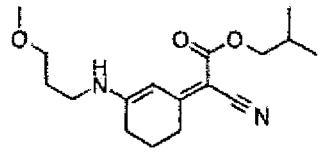<br>(2Z)-cyano-{3-[(3-méthoxy-propyl)amino] cyclohex-2-én-1-ylidène}éthanoate de 2-méthylpropyle | 6. | 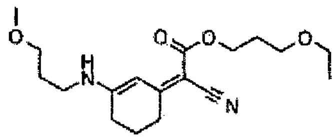<br>(2Z)-cyano-{3-[(3-méthoxy-propyl)amino] cyclohex-2-én-1-ylidène}éthanoate de 3-éthoxypropyle |

**4.** Composition selon la revendication 3, dans laquelle le composé de mérocyanine est le (2Z)-cyano-{3-[(3-méthoxy-propyl)amino]cyclohex-2-én-1-ylidène}éthanoate de 2-éthoxyéthyle (2) selon sa configuration géométrique E/Z, répondant à la structure suivante :

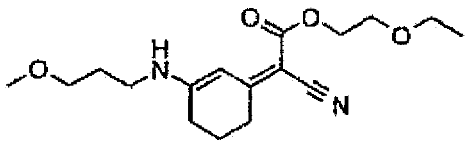

et/ou selon sa configuration géométrique E/E, répondant à la structure suivante :

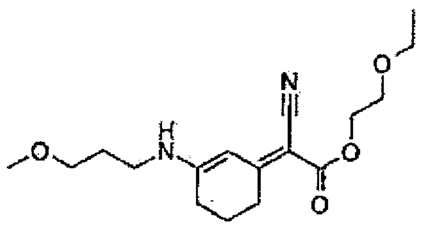

**5.** Composition selon l'une quelconque des revendications 1 à 4, dans laquelle le ou les composés de mérocyanine de formule (1) sont présents selon une concentration allant de 0,1% à 10% en poids et de préférence de 0,2% à 5% en poids, par rapport au poids total de la composition.

**6.** Composition selon l'une quelconque des revendications 1 à 5, dans laquelle l'agent anti-UV organique insoluble se trouve sous la forme de particules ayant une taille moyenne allant de 0,01 à 5 μm, plus préférablement de 0,01 à 2 μm et plus particulièrement de 0,020 à 2 μm.

**7.** Composition selon l'une quelconque des revendications 1 à 6, dans laquelle l'agent anti-UV organique insoluble est choisi parmi les agents anti-UV organiques du type oxalanilide, du type triazine, du type benzotriazole, du type vinylamide, du type cinnamide, du type comprenant un ou plusieurs groupements benzazole et/ou benzofurane, les groupements benzothiophénène ou du type indole, ou du type aryl vinylène cétone, du type dérivé de phénylène bis-benzoxazinone, ou du type dérivé d'amide, de sulfonamide ou de carbamate d'acrylonitrile, ou des mélanges de ceux-ci.

**8.** Composition selon l'une quelconque des revendications 1 à 7, dans laquelle l'agent anti-UV organique insoluble est choisi parmi :

(i) les triazines symétriques substituées par des groupements naphtalényle ou des groupements polyphényle, en particulier la 2,4,6-tris(diphényl)triazine et/ou la 2,4,6-tris(terphényl)triazine ;
(ii) les composés de méthylènebis(hydroxyphénylbenzotriazole) de formule (IV) ci-après

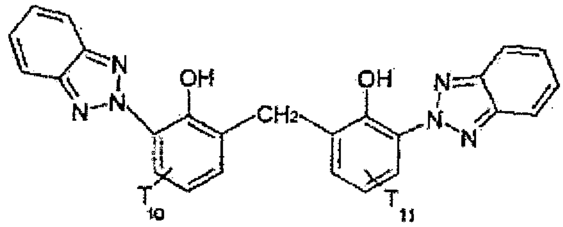

(IV)

où les radicaux $T_{10}$ et $T_{11}$, qui peuvent être identiques ou différents, désignent un radical $C_1$-$C_{18}$-alkyle éventuellement substitué par un ou plusieurs radicaux choisis parmi $C_1$-$C_4$-alkyle et $C_5$-$C_{12}$-cycloalkyle ou un reste aryle ;

(iii) et des mélanges de ceux-ci.

**9.** Composition selon la revendication 8, dans laquelle le composé de méthylènebis(hydroxyphénylbenzotriazole) de formule (IV) se trouve sous la forme d'une dispersion aqueuse de particules ayant une taille de particules moyenne dans la plage allant de 0,01 à 5 µm, plus préférablement de 0,01 à 2 µm et plus particulièrement de 0,020 à 2 µm, en présence d'au moins un agent tensioactif de structure $C_nH_{2n+1}O(C_6H_{10}O_5)_xH$ où n est un nombre entier allant de 8 à 16 et x est le degré moyen de polymérisation de l'unité ($C_6H_{10}O_5$) et va de 1, 4 à 1, 6.

**10.** Composition selon la revendication 8 ou 9, dans laquelle le composé de méthylènebis(hydroxyphénylbenzotriazole) de formule (IV) se trouve sous la forme d'une dispersion aqueuse de particules ayant une taille moyenne allant de 0,02 à 2 µm, plus préférablement de 0,01 à 1,5 µm et plus particulièrement de 0,02 à 1 µm, en présence d'au moins un mono ($C_8$-$C_{20}$) alkylester de polyglycérol ayant un degré de polymérisation du glycérol d'au moins 5.

**11.** Composition selon la revendication 10, dans laquelle le mono ($C_8$-$C_{20}$) alkylester de polyglycérol est choisi parmi le caprate de décaglycérol, le laurate de décaglycérol, le myristate de décaglycérol, l'oléate de décaglycérol, le stéarate de décaglycérol, l'isostéarate de décaglycérol, le caprate d'hexaglycérol, le laurate d'hexaglycérol, le myristate d'hexaglycérol, l'oléate d'hexaglycérol, le stéarate d'hexaglycérol, l'isostéarate d'hexaglycérol, le caprate de pentaglycérol, le laurate de pentaglycérol, le myristate de pentaglycérol, l'oléate de pentaglycérol, le stéarate de pentaglycérol et l'isostéarate de pentaglycérol, et plus particulièrement parmi le caprate de décaglycérol et le laurate de décaglycérol.

**12.** Composition selon la revendication 10 ou 11, dans laquelle la quantité de composé de méthylènebis(hydroxyphénylbenzotriazole) de formule (IV) dans la dispersion aqueuse va de 10% à 50% en poids, et plus préférablement de 30% à 50% en poids, par rapport au poids total de la dispersion.

**13.** Composition selon l'une quelconque des revendications 10 à 12, dans laquelle le rapport pondéral du composé de méthylènebis (hydroxyphénylbenzotriazole) au mono ($C_8$-$C_{30}$) alkylester de polyglycérol va de 0,05 à 0,5, et plus préférablement de 0,1 à 0,3.

**14.** Composition selon l'une quelconque des revendications 9 à 13, dans laquelle le composé de méthylènebis(hydroxyphénylbenzotriazole) de formule (IV) sous forme de dispersion aqueuse est le composé de 2,2'-méthylènebis[6-(2H-benzotriazol-2-yl)-4-(1,1,3,3-tétra-méthylbutyl)phénol] répondant à la structure suivante :

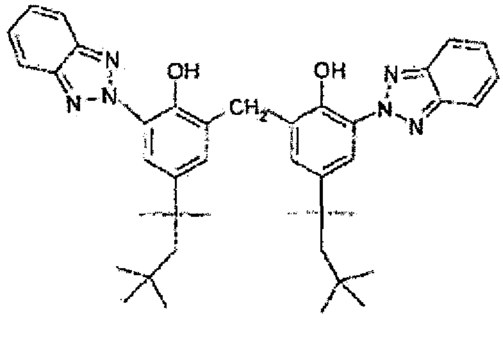

composé (a)

**15.** Composition selon l'une quelconque des revendications 1 à 14, dans laquelle le ou les agents anti-UV organiques

insolubles de l'invention sont présents selon une concentration en matériau actif allant de 0,1% à 15% en poids et plus particulièrement de 0,5% à 10% en poids, par rapport au poids total de la composition.

**16.** Composition selon l'une quelconque des revendications 1 à 5, dans laquelle l'agent anti-UV inorganique insoluble est choisi parmi les pigments d'oxyde métallique revêtus ou non revêtus, choisis en particulier parmi les oxydes de titane, zinc, fer, zirconium et cérium, ou des mélanges de ceux-ci.

**17.** Composition selon la revendication 16, dans laquelle les particules d'oxyde métallique possèdent une taille de particules élémentaires moyenne inférieure ou égale à 0,5 μm, plus préférablement comprise entre 0,005 et 0,5 μm, encore plus préférablement entre 0,01 et 0,2 μm, mieux encore entre 0,01 et 0,1 μm et plus particulièrement préférablement entre 0,015 et 0,05 μm.

**18.** Composition selon la revendication 16 ou 17, dans laquelle l'agent anti-UV minéral insoluble est constitué de particules de dioxyde de titane revêtues ou non revêtues.

**19.** Composition selon l'une quelconque des revendications 1 à 5, et 16 à 18, dans laquelle le ou les agents anti-UV inorganiques insolubles sont présents selon des teneurs allant de 0,1% à 30% en poids, plus particulièrement de 0,5% à 20% en poids, et en particulier de 1% à 10% en poids, par rapport au poids total de la composition.

**20.** Composition telle que définie selon l'une quelconque des revendications précédentes, pour une utilisation dans une méthode de soin et/ou de maquillage d'un matériau kératinique, comprenant l'application de ladite composition à la surface dudit matériau kératinique.

**21.** Composition telle que définie selon l'une quelconque des revendications précédentes, pour une utilisation dans une méthode de limitation de l'assombrissement de la peau et/ou d'amélioration de la couleur et/ou de l'uniformité du teint, comprenant l'application de ladite composition à la surface de la peau.

**22.** Composition telle que définie selon l'une quelconque des revendications précédentes, pour une utilisation dans une méthode de prévention et/ou de traitement des signes de vieillissement d'un matériau kératinique, comprenant l'application de ladite composition à la surface du matériau kératinique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2326405 A
- FR 2440933 A
- EP 0114607 A
- US 5869030 A
- EP 0518773 A
- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071582 A
- US 4749643 A
- GB 2303549 A
- EP 893119 A
- WO 9522959 A
- WO 9703642 A
- GB 2286774 A
- EP 743309 A
- WO 9822447 A
- GB 2319523 A
- EP 0790243 A
- WO 9825922 A
- US 6225467 B
- WO 2004085412 A
- WO 06035000 A
- WO 06034982 A
- WO 06034991 A
- WO 06035007 A
- WO 2006034992 A
- WO 2006034985 A
- US 5687521 A
- US 5373037 A
- US 5362881 A
- US 5237071 A
- US 5166355 A
- DE 19726184
- US 5888481 A
- DE 676103
- CH 350763
- US 5501850 A
- US 5961960 A
- EP 0669323 A
- US 5518713 A
- US 2463264 A
- EP 0921126 A
- EP 0712855 A
- JP 04134042 B
- JP 04134043 B
- EP 0576974 A
- FR 2395023
- JP 01158090 A
- EP 0390683 A
- JP 04134041 B
- FR 2506156
- EP 0693471 A
- FR 2528420
- FR 2529887
- EP 0694521 A
- FR 2638354
- EP 0714880 A
- JP 04290882 B
- FR 2639347 A
- JP 87166517 B
- WO 9310753 A
- WO 9311095 A
- WO 9505150 A
- WO 2007071584 A
- WO 2009063392 A
- WO 2007068371 A
- WO 2008155059 A
- US 5624663 A
- EP 669323 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- EP 0841341 A
- WO 9206778 A
- FR 2315991
- FR 2416008
- US 4077441 A
- US 4850517 A

**Non-patent literature cited in the description**


- *IP.com Journal,* 2009, vol. 9 (5A), 29-30
- Symmetrical Triazine Derivatives. IP.COM Journal. IP.COM INC, 20 September 2004
- *J. Am. Chem. Soc.,* 1957, vol. 79, 5706-5708
- *J. Am. Chem. Soc.,* 1960, vol. 82, 609-5, 611
- *J. Am. Chem. Soc.,* 1960, vol. 82, 609
- *J. Am. Chem. Soc.,* 1957, vol. 79, 5706-5708
- *J. Chim. Phys.,* 1967, vol. 64, 1602
- **E. MARIANI et al.** *16th IFSCC Congress,* 1990
- *CHEMICAL ABSTRACTS,* 919803-06-8
- Symmetrical Triazine Derivatives. IP.COM IPCOM000031257 Journal. INC, 20 September 2004
- McCutcheon's Emulsifiers & Detergents. MC Publishing Company, 1998
- **BANGHAM ; STANDISH ; WATKINS.** *J. Mol. Biol.,* 1965, vol. 13, 238
- **B.L. DIFFEY.** *J. Soc. Cosmet. Chem.,* 1989, vol. 40, 127-133